# EUROPEAN PATENT SPECIFICATION

(11) **EP 1 898 950 B1**
(45) Date of publication and mention of the grant of the patent: **24.11.2010**
(21) Application number: 06799955.7
(22) Date of filing: 20.06.2006
(51) Int. Cl.: G01N 33/574, G01N 33/68, A61K 39/395

(54) **GALK1S AS MODIFIERS OF THE PTEN/AKT PATHWAY**
GALK1S ALS MODIFIKATOREN DES PTEN/AKT-WEGS
GALK1S MODIFICATEURS DE LA VOIE PTEN/AKT

(30) Priority: 20.06.2005 US 692342 P
(43) Date of publication of application: 19.03.2008
(73) Proprietor: Exelixis, Inc., South San Francisco, CA 94083-0511 (US)
(72) Inventor: BRACE, Arthur, Redwood City, CA 94065 (US); BJERKE, Lynn M. Cancer Res., UK Ctr. for Cancer, 15 Cotswold Road Sutton, Surrey SM2 5NG (GB); NIETO-BERGMAN, Susana, Berkeley, CA 94702 (US); FRIEDMAN, Lori S., San Carlos, CA 94070 (US); WARD, Kevin, San Francisco, CA 94110 (US); BLAKE, Robert A., San Carlos, CA 94070 (US)
(74) Representative: Main, Malcolm Charles
(86) International application number: PCT/US2006/024053
(87) International publication number: WO 2007/002131

(56) References cited:
- WO-A-02/04657
- WO-A-2004/078783
- WO-A-2005/002418
- US-A1- 2002 150 954
- DATABASE Geneseq [Online] 26 February 2004 (2004-02-26), "Human hepatic-fibrosis disease marker protein SEQ ID 372." XP002486587 retrieved from EBI accession no. GSP:ADF90910 Database accession no. ADF90910
- THOMPSON JAMES E ET AL: "Putting the rap on Akt." JOURNAL OF CLINICAL ONCOLOGY : OFFICIAL JOURNAL OF THE AMERICAN SOCIETY OF CLINICAL ONCOLOGY 15 OCT 2004, vol. 22, no. 20, 15 October 2004 (2004-10-15), pages 4217-4226, XP002486584 ISSN: 0732-183X
- PAEZ JUAN ET AL: "PI3K/PTEN/AKT pathway. A critical mediator of oncogenic signaling." CANCER TREATMENT AND RESEARCH 2003, vol. 115, 2003, pages 145-167, XP002486585 ISSN: 0927-3042
- THOMPSON J.E. ET AL.: 'Putting the Rap on Akt' J. CLIN. ONCOL. vol. 22, no. 20, 15 October 2004, pages 4217 - 4226, XP002486584
- SANGIUOLO F. ET AL.: 'Biochemical Characterization of Two GALK1 Mutations in Patients with Galactokinase Deficiency' HUM. MUTAT. vol. 23, no. 4, April 2004, pages 396 - 312, XP008125321

## Description

### BACKGROUND OF THE INVENTION

Intracellular levels of phosphorylation are regulated by the coordinated action of protein kinases and phosphatases. Somatic mutations in the PTEN (Phosphatase and Tensin homolog deleted on chromosome 10) gene are known to cause tumors in a variety of human tissues. In addition, germline mutations in PTEN are the cause of human diseases (Cowden disease and Bannayan-Zonana syndrome) associated with increased risk of breast and thyroid cancer (Nelen MR et al. (1997) Hum Mol Genet, 8:1383-1387; Liaw D et al. (1997) Nat Genet, 1:64-67; Marsh DJ et al. (1998) Hum Mol Genet, 3:507-515). PTEN acts as a tumor suppressor by regulating several signaling pathways through the second messenger phosphatidylinositol 3,4,5 triphosphate (PIP3). PTEN dephosphorylates the D3 position of PIP3 and downregulates signaling events dependent on PIP3 levels (Maehama T and Dixon JE (1998) J Biol Chem, 22, 13375-8). This inhibits downstream targets mainly protein kinase B (PKB/AKT). PTEN sequence is conserved in evolution, and exists in mouse (Hansen GM and Justice MJ (1998) Mamm Genome, 9(1):88-90), Drosophila (Goberdhan DC et al (1999) Genes and Dev, 24:3244-58; Huang H et al (1999) Development 23:5365-72), and C. elegans (Ogg S and Ruvkun G, (1998) Mol Cell, (6):887-93). Studies in these model organisms have helped to elucidate the role of PTEN in processes relevant to tumorigenesis. In Drosophila, the PTEN homolog (dPTEN) has been shown to regulate cell size, survival, and proliferation (Huang et al, supra; Goberdhan et al, supra; Gao X et al, 2000, 221:404-418). In mice, loss of PTEN function increases cancer susceptibility (Di Cristofano A et al (1998) Nature Genetics, 19:348-355; Suzuki A et al (1998) Curr. Biol., 8:1169-78).

AKT signaling is frequently hyperactivated by a variety of mechanisms in a wide range of human cancers, including melanoma, breast, lung, prostate, and ovarian tumors (see Vivanco I and Sawyers CL (2002) Nat Rev Cancer. 2(7):489-501; Scheid MPand Woodgett JR (2001) J Mammary Gland Biol Neoplasia. 6(1):83-99). In tumor cells, the AKT protein kinase activity can be elevated by amplification and overexpression of the AKT2 gene, or by increased production of phosphatidylinositol (3, 4, 5) trisphosphate (PIP3), which activates AKT by recruitment to the plasma membrane. In normal phosphoinositide metabolism, phosphatidylinositol (3, 4) bisphosphate (PIP2) is phosphorylated by phosphatidylinositol 3-kinase (PI3K) to generate PIP3, and PIP3 is dephosphorylated back to PIP2 by the lipid phosphatase PTEN. Most commonly, however, PIP3 levels in tumor cells are elevated by mutation or deletion of the PTEN tumor suppressor, at rates as high as 40-50% of prostate cancers.

The PTEN/AKT pathway promotes tumor progression by enhancing cell proliferation, growth, survival, and motility, and by suppressing apoptosis. These effects are mediated by several AKT substrates, including the related transcription factors FKHR and AFX, for which phosphorylation by AKT mediates nuclear export.

Signaling through the TOR (mTOR) branch of the PTEN/AKT signaling pathway regulates protein synthesis, which is directly involved in the growth activation and cellular transformation properties of AKT signaling. TOR directly phosphorylates several targets including 4EBP1 and p70S6 kinase. p70S6 kinase directly phosphorylates ribosomal protein S6 (RPS6) (Bader AG et al. (2004) Oncogene 23:3145-3150; Hay N et al. (2004) Genes Dev. 18:1926-1945). Additional direct AKT substrates have been identified which can serve as a readout for PTEN/AKT signaling activity, including the protein PRAS40 (Kovacina KS et al. (2003) JBC 278(12):10189-10194).

Identification of the involvement of novel genes in particular pathways, such as disease pathways, and their function in such pathways can directly contribute to the understanding of modulation of these pathways. Further, the identified genes may be attractive candidate targets for novel therapeutics.

WO 2004/078783 describes an assay for identifying early regulatory events occurring in apoptosis and, in particular, in the inhibition of apoptosis by GM- CSF. US2002/150954 describes methods for the identification, biochemical characterisation and therapeutic use of agents which impact PTEN, p53, PI-kinase and AKT-mediated cellular signalling.

### SUMMARY OF THE INVENTION

We have discovered genes that modify the PTEN/AKT pathway in human cells, hereinafter referred to as Modifier of PTEN/AKT (MPTENAKT). Specifically, we have discovered that one gene, the Galactose Kinase 1 gene (GALK1) modifies PTEN/AKT pathway in a number of human tissues and human cell lines. GALK1 converts galactose into galactose-1-phosphate which then feeds into glycolysis. There is homology between GALK1 and other GHMP kinases between approximately amino acids 300 and 360. GALK1 may function as the cellular glactose sensor. Mutations in GALK1 cause galactosemia. GALK1 is only 30% identical to GALK2. GALK1 mRNA is over-expressed in colon, head/neck, lung, ovary, pancreas, skin and stomach tumors. GALK1 is well expressed in standard tumor cell lines including MDA-MB231T (breast tumor cell line), A549 (non-small cell lung tumor cell line), U87MG (glioblastoma cell line) and PC-3 (prostate tumor cell line).

Described are methods for utilizing these PTEN/AKT modifier genes and polypeptides to identify GALK1-modulating agents that are candidate therapeutic agents that can be used in the treatment of disorder associated with defective or impaired PTEN/AKT function and/or GALS1 function.
The invention provides an in vitro method of identifying a candidate PTEN/AKT modulating agent, said method comprising the steps of:
(a) providing an assay system capable of detecting GALK1 expression and/or kinase activity comprising a GALK I polypeptide or a nucleic acid encoding said polypeptide;
   wherein said GALK1 polypeptide comprises:
   (i) the polypeptide having the amino acid sequence shown as SEQ ID NO: 1;
   (ii) a functionally active fragment of the polypeptide having the amino acid sequence shown as SEQ ID NO: 1, wherein said functionally active fragment is a kinase domain-containing fragment having galactose kinase activity and comprising at least 100 contiguous amino acids of SEQ ID NO: 1; or
   (iii) a polypeptide which has at least 80% sequence identity with SEQ ID NO: 1 and has galactose kinase activity;
(b) contacting the assay system with a test agent; and
(c) determining the expression or kinase activity of GALK1 in the assay system, wherein a change in GALK1 expression or kinase activity between the presence and absence of said test agent identifies the test agent as a candidate PTEN/AKT modulating agent.
Preferred GALK1-modulating agents specifically bind to GALK1 polypeptides and restore PTEN/AKT function. Other preferred GALK1-modulating agents are nucleic acid modulators such as antisense oligomers and RNAi that repress GALK1 gene expression or product activity by, for example, binding to and inhibiting the respective nucleic acid (i.e. DNA or mRNA).

GALK1 modulating agents may be evaluated by any convenient *in vitro* assay for molecular interaction with an GALK1 polypeptide or nucleic acid. Candidate GALK1 modulating agents are tested with an assay system comprising a GALK1 polypeptide or nucleic acid. Agents that produce a change in the activity of the assay system relative to controls are identified as candidate PTEN/AKT modulating agents. The assay system may be cell-based or cell-free. GALK1-modulating agents include GALK1 related proteins (e.g. dominant negative mutants, and biotherapeutics); GALK1 -specific antibodies; GALK1 - specific antisense oligomers and other nucleic acid modulators; and chemical agents that specifically bind to or interact with GALK1 or compete with GALK1 binding partner (e.g. by binding to an GALK1 binding partner). In one specific embodiment, a small molecule modulator is identified using a binding assay. In specific embodiments, the screening assay system is selected from an apoptosis assay, a cell proliferation assay, an angiogenesis assay, and a hypoxic induction assay.

In another embodiment, candidate PTEN/AKT pathway modulating agents are further tested using a second assay system that detects changes in the PTEN/AKT pathway, such as angiogenic, apoptotic, or cell proliferation changes produced by the originally identified candidate agent or an agent derived from the original agent. The second assay system may use cultured cells

### DETAILED DESCRIPTION OF THE INVENTION

We designed a genetic screen to identify suppressors genes that when inactivated, decrease signaling through the PTEN/AKT pathway. Several genes were identified. Accordingly, these GALK1 genes (i.e., nucleic acids and polypeptides) are attractive drug targets for the treatment of pathologies associated with a defective PTEN/AKT signaling pathway, such as cancer. Table 1 (Example II) lists these genes.

In vitro methods of assessing GALK1 function are provided herein. Modulation of the GALK1 or their respective binding partners is useful for understanding the association of the PTEN/AKT pathway and its members in normal and disease conditions and for developing diagnostics and therapeutic modalities for PTEN/AKT related pathologies. GALK1-modulating agents that act by inhibiting or enhancing GALK1 expression, directly or indirectly, for example, by affecting an GALK1 function such as enzymatic (e.g., catalytic) or binding activity, can be identified using methods provided herein. GALK1 modulating agents are useful in diagnosis, therapy and pharmaceutical development.

### Nucleic acids and polypeptides of the invention

Sequences related to GALK1 nucleic acids and polypeptides that can be used in the invention are disclosed in Genbank (referenced by Genbank identifier (GI) or RefSeq number), shown in Table 1 and in the appended sequence listing.

The term "GALK1 polypeptide" refers to a full-length GALK1 protein or a functionally active fragment or derivative thereof. A "functionally active" GALK1 fragment or derivative exhibits functional galactose kinase activity associated with a full-length, wild-type GALK1 protein.
The functional activity of GALK1 proteins, derivatives and fragments can be assayed by various methods known to one skilled in the art (Current Protocols in Protein Science (1998) Coligan et al., eds., John Wiley & Sons, Inc., Somerset, New Jersey) and as further discussed below. In one embodiment, a functionally active GALK1 polypeptide is an GALK1 derivative capable of rescuing defective endogenous GALK1 activity, such as in cell based or animal assays; the rescuing derivative may be from the same or a different species. For purposes herein, functionally active fragments comprise one or more kinase domains of an GALK1. Protein domains can be identified using the PFAM program (Bateman A., et al., Nucleic Acids Res, 1999, 27:260-2). Methods for obtaining GALK1 polypeptides are also further described below. Fragments are functionally active, domain-containing fragments comprising at least 100 contiguous amino acids of an GALK1. In further preferred embodiments, the fragment comprises the entire functionally active domain.

The term "GALK1 nucleic acid" refers to a DNA or RNA molecule that encodes an GALK1 polypeptide. Preferably, the GALK1 polypeptide or nucleic acid or fragment thereof is from a human, but can also be an ortholog, or derivative thereof with at least 80%, more preferably 85%, still more preferably 90%, and most preferably at least 95% sequence identity with human GALK1. As used herein, "percent (%) sequence identity" with respect to a subject sequence, or a specified portion of a subject sequence, is defined as the percentage of nucleotides or amino acids in the candidate derivative sequence identical with the nucleotides or amino acids in the subject sequence (or specified portion thereof), after aligning the sequences and introducing gaps, if necessary to achieve the maximum percent sequence identity, as generated by the program WU-BLAST-2.0a19 (Altschul et al., J. Mol. Biol. (1997) 215:403-410) with all the search parameters set to default values. The HSP S and HSP S2 parameters are dynamic values and are established by the program itself depending upon the composition of the particular sequence and composition of the particular database against which the sequence of interest is being searched. A % identity value is determined by the number of matching identical nucleotides or amino acids divided by the sequence length for which the percent identity is being reported. "Percent (%) amino acid sequence similarity" is determined by doing the same calculation as for determining % amino acid sequence identity, but including conservative amino acid substitutions in addition to identical amino acids in the computation.

A conservative amino acid substitution is one in which an amino acid is substituted for another amino acid having similar properties such that the folding or activity of the protein is not significantly affected. Aromatic amino acids that can be substituted for each other are phenylalanine, tryptophan, and tyrosine; interchangeable hydrophobic amino acids are leucine, isoleucine, methionine, and valine; interchangeable polar amino acids are glutamine and asparagine; interchangeable basic amino acids are arginine, lysine and histidine; interchangeable acidic amino acids are aspartic acid and glutamic acid; and interchangeable small amino acids are alanine, serine, threonine, cysteine and glycine.

Alternatively, an alignment for nucleic acid sequences is provided by the local homology algorithm of Smith and Waterman (Smith and Waterman, 1981, Advances in Applied Mathematics 2:482-489; database: European Bioinformatics Institute; Smith and Waterman, 1981, J. of Molec.Biol., 147:195-197; Nicholas et al., 1998, "A Tutorial on Searching Sequence Databases and Sequence Scoring Methods" (www.psc.edu) and references cited therein.; W.R. Pearson, 1991, Genomics 11:635-650). This algorithm can be applied to amino acid sequences by using the scoring matrix developed by Dayhoff (Dayhoff: Atlas of Protein Sequences and Structure, M. O. Dayhoff ed., 5 suppl. 3:353-358, National Biomedical Research Foundation, Washington, D.C., USA), and normalized by Gribskov (Gribskov 1986 Nucl. Acids Res. 14(6):6745-6763). The Smith-Waterman algorithm may be employed where default parameters are used for scoring (for example, gap open penalty of 12, gap extension penalty of two). From the data generated, the "Match" value reflects "sequence identity."

Derivative nucleic acid molecules of the subject nucleic acid molecules include sequences that hybridize to the nucleic acid sequence of an GALK1. The stringency of hybridization can be controlled by temperature, ionic strength, pH, and the presence of denaturing agents such as formamide during hybridization and washing. Conditions routinely used are set out in readily available procedure texts (*e.g*., Current Protocol in Molecular Biology, Vol. 1, Chap. 2.10, John Wiley & Sons, Publishers (1994); Sambrook et al., Molecular Cloning, Cold Spring Harbor (1989)). In some embodiments, a nucleic acid molecule of the invention is capable of hybridizing to a nucleic acid molecule containing the nucleotide sequence of an GALK1 under high stringency hybridization conditions that are: prehybridization of filters containing nucleic acid for 8 hours to overnight at 65° C in a solution comprising 6X single strength citrate (SSC) (1X SSC is 0.15 M NaCl, 0.015 M Na citrate; pH 7.0), 5X Denhardt's solution, 0.05% sodium pyrophosphate and 100 µg/ml herring sperm DNA; hybridization for 18-20 hours at 65° C in a solution containing 6X SSC, 1X Denhardt's solution, 100 µg/ml yeast tRNA and 0.05% sodium pyrophosphate; and washing of filters at 65° C for 1h in a solution containing 0.1X SSC and 0.1% SDS (sodium dodecyl sulfate).

In other embodiments, moderately stringent hybridization conditions are used that are: pretreatment of filters containing nucleic acid for 6 h at 40° C in a solution containing 35% formamide, 5X SSC, 50 mM Tris-HCl (pH7.5), 5mM EDTA, 0.1% PVP, 0.1% Ficoll, 1% BSA, and 500 µg/ml denatured salmon sperm DNA; hybridization for 18-20h at 40° C in a solution containing 35% formamide, 5X SSC, 50 mM Tris-HCl (pH7.5), 5mM EDTA, 0.02% PVP, 0.02% Ficoll, 0.2% BSA, 100 µg/ml salmon sperm DNA, and 10% (wt/vol) dextran sulfate; followed by washing twice for 1 hour at 55° C in a solution containing 2X SSC and 0.1% SDS.

Alternatively, low stringency conditions can be used that are: incubation for 8 hours to overnight at 37° C in a solution comprising 20% formamide, 5 x SSC, 50 mM sodium phosphate (pH 7.6), 5X Denhardt's solution, 10% dextran sulfate, and 20 µg/ml denatured sheared salmon sperm DNA; hybridization in the same buffer for 18 to 20 hours; and washing of filters in 1 x SSC at about 37° C for 1 hour.

### Isolation, Production, Expression, and Mis-expression of GALK1 Nucleic Acids and Polypeptides

GALK1 nucleic acids and polypeptides are useful for identifying and testing agents that modulate GALK1 function and for other applications related to the involvement of GALK1 in the PTEN/AKT pathway. GALK1 nucleic acids and derivatives and orthologs thereof may be obtained using any available method. For instance, techniques for isolating cDNA or genomic DNA sequences of interest by screening DNA libraries or by using polymerase chain reaction (PCR) are well known in the art. In general, the particular use for the protein will dictate the particulars of expression, production, and purification methods. For instance, production of proteins for use in screening for modulating agents may require methods that preserve specific biological activities of these proteins, whereas production of proteins for antibody generation may require structural integrity of particular epitopes. Expression of proteins to be purified for screening or antibody production may require the addition of specific tags (*e.g*., generation of fusion proteins). Overexpression of an GALK1 protein for assays used to assess GALK1 function, such as involvement in cell cycle regulation or hypoxic response, may require expression in eukaryotic cell lines capable of these cellular activities. Techniques for the expression, production, and purification of proteins are well known in the art; any suitable means therefore may be used (e.g., Higgins SJ and Hames BD (eds.) Protein Expression: A Practical Approach, Oxford University Press Inc., New York 1999; Stanbury PF et al., Principles of Fermentation Technology, 2nd edition, Elsevier Science, New York, 1995; Doonan S (ed.) Protein Purification Protocols, Humana Press, New Jersey, 1996; Coligan JE et al, Current Protocols in Protein Science (eds.), 1999, John Wiley & Sons, New York). In particular embodiments, recombinant GALK1 is expressed in a cell line known to have defective PTEN and/or AKT function. The recombinant cells are used in cell-based screening assay systems of the invention, as described further below.

The nucleotide sequence encoding an GALK1 polypeptide can be inserted into any appropriate expression vector. The necessary transcriptional and translational signals, including promoter/enhancer element, can derive from the native GALK1 gene and/or its flanking regions or can be heterologous. A variety of host-vector expression systems may be utilized, such as mammalian cell systems infected, with virus (*e.g*. vaccinia virus, adenovirus, etc.); insect cell systems infected with virus (e.g. baculovirus); microorganisms such as yeast containing yeast vectors, or bacteria transformed with bacteriophage, plasmid, or cosmid DNA. An isolated host cell strain that modulates the expression of, modifies, and/or specifically processes the gene product may be used.

To detect expression of the GALK1 gene product, the expression vector can comprise a promoter operably linked to an GALK1 gene nucleic acid, one or more origins of replication, and, one or more selectable markers (*e.g*. thymidine kinase activity, resistance to antibiotics, etc.). Alternatively, recombinant expression vectors can be identified by assaying for the expression of the GALK1 gene product based on the physical or functional properties of the GALK1 protein in *in vitro* assay systems (*e.g*. immunoassays).

The GALK1 protein, fragment, or derivative may be optionally expressed as a fusion, or chimeric protein product (i.e. it is joined via a peptide bond to a heterologous protein sequence of a different protein), for example to facilitate purification or detection. A chimeric product can be made by ligating the appropriate nucleic acid sequences encoding the desired amino acid sequences to each other using standard methods and expressing the chimeric product. A chimeric product may also be made by protein synthetic techniques, *e.g*. by use of a peptide synthesizer (Hunkapiller et al., Nature (1984) 310:105-111).

Once a recombinant cell that expresses the GALK1 gene sequence is identified, the gene product can be isolated and purified using standard methods (*e.g.* ion exchange, affinity, and gel exclusion chromatography; centrifugation; differential solubility; electrophoresis). Alternatively, native GALK1 proteins can be purified from natural sources, by standard methods (*e.g*. immunoaffinity purification). Once a protein is obtained, it may be quantified and its activity measured by appropriate methods, such as immunoassay, bioassay, or other measurements of physical properties, such as crystallography.

The methods of this invention may also use cells that have been engineered for altered expression (mis-expression) of GALK1 or other genes associated with the PTEN/AKT pathway. As used herein, mis-expression encompasses ectopic expression, over-expression, under-expression, and non-expression (*e.g*. by gene knock-out or blocking expression that would otherwise normally occur).

### Genetically modified animals

Animal models that have been genetically modified to alter GALK1 expression may be used in *in vivo* assays to test for activity of a candidate PTEN/AKT modulating agent, or to further assess the role of GALK1 in a PTEN/AKT pathway process such as apoptosis or cell proliferation. The altered GALK1 expression may result in a detectable phenotype, such as decreased or increased levels of cell proliferation, angiogenesis, or apoptosis compared to control animals having normal GALK1 expression. The genetically modified animal may additionally have altered PTEN and/or AKT expression (e.g. PTEN and/or AKT knockout). Genetically modified animals may be mammals such as primates, rodents (for example mice or rats), among others. Non-mammalian species include zebrafish, *C. elegans,* and *Drosophila.* Particular genetically modified animals are transgenic animals having a heterologous nucleic acid sequence present as an extrachromosomal element in a portion of its cells, i.e. mosaic animals (see, for example, techniques described by Jakobovits, 1994, Curr. Biol. 4:761-763.) or stably integrated into its germ line DNA (i.e., in the genomic sequence of most or all of its cells). Heterologous nucleic acid is introduced into the germ line of such transgenic animals by genetic manipulation of, for example, embryos or embryonic stem cells of the host animal.

Methods of making transgenic animals are well-known in the art (for transgenic mice see Brinster et al., Proc. Nat. Acad. Sci. USA 82: 4438-4442 (1985), U.S. Pat. Nos. 4,736,866 and 4,870,009, both by Leder et al., U.S. Pat. No. 4,873,191 by Wagner et al., and Hogan, B., Manipulating the Mouse Embryo, Cold Spring Harbor Laboratory Press, Cold Spring Harbor, N.Y., (1986); for particle bombardment see U.S. Pat. No., 4,945,050, by Sandford et al.; for transgenic *Drosophila* see Rubin and Spradling, Science (1982) 218:348-53 and U.S. Pat. No. 4,670,388; for transgenic insects see Berghammer A.J. et al., A Universal Marker for Transgenic Insects (1999) Nature 402:370-371; for transgenic Zebrafish see Lin S., Transgenic Zebrafish, Methods Mol Biol. (2000);136:375-3830); for microinjection procedures for fish, amphibian eggs and birds see Houdebine and Chourrout, Experientia (1991) 47:897-905; for transgenic rats see Hammer et al., Cell (1990) 63:1099-1112; and for culturing of embryonic stem (ES) cells and the subsequent production of transgenic animals by the introduction of DNA into ES cells using methods such as electroporation, calcium phosphate/DNA precipitation and direct injection see, e.g., Teratocarcinomas and Embryonic Stem Cells, A Practical Approach, E. J. Robertson, ed., IRL Press (1987)). Clones of the nonhuman transgenic animals can be produced according to available methods (see Wilmut, I. et al. (1997) Nature 385:810-813; and PCT International Publication Nos. WO 97/07668 and WO 97/07669).

A transgenic animal may be a "knock-out" animal having a heterozygous or homozygous alteration in the sequence of an endogenous GALK1 gene that results in a decrease of GALK1 function, preferably such that GALK1 expression is undetectable or insignificant. Knock-out animals are typically generated by homologous recombination with a vector comprising a transgene having at least a portion of the gene to be knocked out. Typically a deletion, addition or substitution has been introduced into the transgene to functionally disrupt it. The transgene can be a human gene (e.g., from a human genomic clone) but more preferably is an ortholog of the human gene derived from the transgenic host species. For example, a mouse GALK1 gene is used to construct a homologous recombination vector suitable for altering an endogenous GALK1 gene in the mouse genome. Detailed methodologies for homologous recombination in mice are available (see Capecchi, Science (1989) 244:1288-1292; Joyner et al., Nature (1989) 338:153-156). Procedures for the production of non-rodent transgenic mammals and other animals are also available (Houdebine and Chourrout, *supra*; Pursel et al., Science (1989) 244:1281-1288; Simms et al., Bio/Technology (1988) 6:179-183). Knock-out animals, such as mice harboring a knockout of a specific gene, may be used to produce antibodies against the human counterpart of the gene that has been knocked out (Claesson MH et al., (1994) Scan J Immunol 40:257-264; Declerck PJ et al., (1995) J Biol Chem. 270:8397-400).

A transgenic animal may be a "knock-in" animal having an alteration in its genome that results in altered expression (e.g., increased (including ectopic) or decreased expression) of the GALK1 gene, e.g., by introduction of additional copies of GALK1, or by operatively inserting a regulatory sequence that provides for altered expression of an endogenous copy of the GALK1 gene. Such regulatory sequences include inducible, tissue-specific, and constitutive promoters and enhancer elements. The knock-in can be homozygous or heterozygous.

Transgenic nonhuman animals can also be produced that contain selected systems allowing for regulated expression of the transgene. One example of such a system that may be produced is the cre/loxP recombinase system of bacteriophage P1 (Lakso et al., PNAS (1992) 89:6232-6236; U.S. Pat. No. 4,959,317). If a cre/loxP recombinase system is used to regulate expression of the transgene, animals containing transgenes encoding both the Cre recombinase and a selected protein are required. Such animals can be provided through the construction of "double" transgenic animals, e.g., by mating two transgenic animals, one containing a transgene encoding a selected protein and the other containing a transgene encoding a recombinase. Another example of a recombinase system is the FLP recombinase system of Saccharomyces cerevisiae (O'Gorman et al. (1991) Science 251:1351-1355; U.S. Pat. No. 5,654,182). Both Cre-LoxP and Flp-Frt may be used in the same system to regulate expression of the transgene, and for sequential deletion of vector sequences in the same cell (Sun X et al (2000) Nat Genet 25:83-6).

The genetically modified animals can be used in genetic studies to further elucidate the PTEN/AKT pathway, as animal models of disease and disorders implicating defective PTEN/AKT function, and for *in vivo* testing of candidate therapeutic agents, such as those identified in screens described below. The candidate therapeutic agents are administered to a genetically modified animal having altered GALK1 function and phenotypic changes are compared with appropriate control animals such as genetically modified animals that receive placebo treatment, and/or animals with unaltered GALK1 expression that receive candidate therapeutic agent.

In addition to the above-described genetically modified animals having altered GALK1 function, animal models having defective PTEN/AKT function (and otherwise normal GALK1 function), can be used. For example, a mouse with defective PTEN or AKT function can be used to assess, in vivo, the activity of a candidate PTEN/AKT modulating agent identified in one of the in vitro assays described below. Transgenic mice with defective PTEN function have been described in literature (Di Cristofano et al, supra). Transgenic mice with defective AKT function have also been described (Chen, W. S. et al (2001) Genes Dev. 15: 2203-2208; Condorelli, G. et al (2002) Proc. Nat. Acad. Sci. 99: 12333-12338; Peng, X. et al (2003) Genes Dev. 17: 1352-1365). Preferably, the candidate PTEN/AKT modulating agent when administered to a model system with cells defective in PTEN/AKT function, produces a detectable phenotypic change in the model system indicating that the PTEN/AKT function is restored, i.e., the cells exhibit normal cell cycle progression.

### Modulating Agents

The invention provides methods to identify agents that interact with and/or modulate the function of GALK1 and/or the PTEN/AKT pathway. Such agents are useful in a variety of diagnostic and therapeutic applications associated with the PTEN/AKT pathway, as well as in further analysis of the GALK1 protein and its contribution to the PTEN/AKT pathway.

As used herein, an "GALK1-modulating agent" is any agent that modulates GALK1 function, for example, an agent that interacts with GALK1 to inhibit or enhance GALK1 activity or otherwise affect normal GALK1 function. GALK1 function can be affected at any level, including transcription, protein expression, protein localization, and cellular or extra-cellular activity. In a preferred embodiment, the GALK1 - modulating agent specifically modulates the function of the GALK1. The phrases "specific modulating agent", "specifically modulates", etc., are used herein to refer to modulating agents that directly bind to the GALK1 polypeptide or nucleic acid, and preferably inhibit, enhance, or otherwise alter, the function of the GALK1. These phrases also encompass modulating agents that alter the interaction of the GALK1 with a binding partner, substrate, or cofactor (e.g. by binding to a binding partner of an GALK1, or to a protein/binding partner complex, and altering GALK1 function). The GALK1- modulating agent is a modulator of the PTEN/AKT pathway (e.g. it restores and/or upregulates PTEN and/or AKT function) and thus is also a PTEN/AKT-modulating agent.

Preferred GALK1-modulating agents include small molecule compounds; GALK1-interacting proteins, including antibodies and other biotherapeutics; and nucleic acid modulators such as antisense and RNA inhibitors. The modulating agents may be formulated in pharmaceutical compositions, for example, as compositions that may comprise other active ingredients, as in combination therapy, and/or suitable carriers or excipients. Techniques for formulation and administration of the compounds may be found in "Remington's Pharmaceutical Sciences" Mack Publishing Co., Easton, PA, 19^{th} edition.

### Small molecule modulators

Small molecules are often preferred to modulate function of proteins with enzymatic function, and/or containing protein interaction domains. Chemical agents, referred to in the art as "small molecule" compounds are typically organic, non-peptide molecules, having a molecular weight up to 10,000, preferably up to 5,000, more preferably up to 1,000, and most preferably up to 500 daltons. This class of modulators includes chemically synthesized molecules, for instance, compounds from combinatorial chemical libraries. Synthetic compounds may be rationally designed or identified based on known or inferred properties of the GALK1 protein or may be identified by screening compound libraries. Alternative appropriate modulators of this class are natural products, particularly secondary metabolites from organisms such as plants or fungi, which can also be identified by screening compound libraries for GALK1-modulating activity. Methods for generating and obtaining compounds are well known in the art (Schreiber SL, Science (2000) 151: 1964-1969; Radmann J and Gunther J, Science (2000) 151:1947-1948).

Small molecule modulators identified from screening assays, as described below, can be used as lead compounds from which candidate clinical compounds may be designed, optimized, and synthesized. Such clinical compounds may have utility in treating pathologies associated with the PTEN/AKT pathway. The activity of candidate small molecule modulating agents may be improved several-fold through iterative secondary functional validation, as further described below, structure determination, and candidate modulator modification and testing. Additionally, candidate clinical compounds are generated with specific regard to clinical and pharmacological properties. For example, the reagents may be derivatized and rescreened using *in vitro* and *in vivo* assays to optimize activity and minimize toxicity for pharmaceutical development.

### Protein Modulators

Specific GALK1-interacting proteins are useful in a variety of diagnostic and therapeutic applications related to the PTEN/AKT pathway and related disorders, as well as in validation assays for other GALK1-modulating agents.
GALK1-interacting proteins affect normal GALK1 function, including transcription, protein expression, protein localization, and cellular or extra-cellular activity. In another embodiment, GALK1-interacting proteins are useful in detecting and providing information about the function of GALK1 proteins, as is relevant to PTEN/AKT related disorders, such as cancer (e.g., for diagnostic means).

An GALK1-interacting protein may be endogenous, i.e. one that naturally interacts genetically or biochemically with an GALK1, such as a member of the GALK1 pathway that modulates GALK1 expression, localization, and/or activity. GALK1-modulators include dominant negative forms of GALK1-interacting proteins and of GALK1 proteins themselves. Yeast two-hybrid and variant screens offer preferred methods for identifying endogenous GALK1-interacting proteins (Finley, R. L. et al. (1996) in DNA Cloning-Expression Systems: A Practical Approach, eds. Glover D. & Hames B. D (Oxford University Press, Oxford, England), pp. 169-203; Fashema SF et al., Gene (2000) 250:1-14; Drees BL Curr Opin Chem Biol (1999) 3:64-70; Vidal M and Legrain P Nucleic Acids Res (1999) 27:919-29; and U.S. Pat. No. 5,928,868). Mass spectrometry is an alternative preferred method for the elucidation of protein complexes (reviewed in, e.g., Pandley A and Mann M, Nature (2000) 405:837-846; Yates JR 3rd, Trends Genet (2000) 16:5-8).

An GALK1-interacting protein may be an exogenous protein, such as an GALK1-specific antibody or a T-cell antigen receptor (see, e.g., Harlow and Lane (1988) Antibodies, A Laboratory Manual, Cold Spring Harbor Laboratory; Harlow and Lane (1999) Using antibodies: a laboratory manual. Cold Spring Harbor, NY: Cold Spring Harbor Laboratory Press). GALK1 antibodies are further discussed below.

In preferred embodiments, an GALK1-interacting protein specifically binds an GALK1 protein. In alternative preferred embodiments, an GALK1-modulating agent binds an GALK1 substrate, binding partner, or cofactor.

### Antibodies

In another embodiment, the protein modulator is an GALK1 specific antibody agonist or antagonist. The antibodies have therapeutic and diagnostic utilities, and can be used in screening assays to identify GALK1 modulators. The antibodies can also be used in dissecting the portions of the GALK1 pathway responsible for various cellular responses and in the general processing and maturation of the GALK1.

Antibodies that specifically bind GALK1 polypeptides can be generated using known methods. Preferably the antibody is specific to a mammalian ortholog of GALK1 polypeptide, and more preferably, to human GALK1. Antibodies may be polyclonal, monoclonal (mAbs), humanized or chimeric antibodies, single chain antibodies, Fab fragments, F(ab').sub.2 fragments, fragments produced by a FAb expression library, anti-idiotypic (anti-Id) antibodies, and epitope-binding fragments of any of the above. Epitopes of GALK1 which are particularly antigenic can be selected, for example, by routine screening of GALK1 polypeptides for antigenicity or by applying a theoretical method for selecting antigenic regions of a protein (Hopp and Wood (1981), Proc. Nati. Acad. Sci. U.S.A. 78:3824-28; Hopp and Wood, (1983) Mol. Immunol. 20:483-89; Sutcliffe et al., (1983) Science 219:660-66) to the amino acid sequence of an GALK1. Monoclonal antibodies with affinities of 10⁸ M⁻¹ preferably 10⁹ M⁻¹ to 10¹⁰ M⁻¹, or stronger can be made by standard procedures as described (Harlow and Lane, *supra*; Goding (1986) Monoclonal Antibodies: Principles and Practice (2d ed) Academic Press, New York; and U.S. Pat. Nos. 4,381,292; 4,451,570; and 4,618,577). Antibodies may be generated against crude cell extracts of GALK1 or substantially purified fragments thereof. If GALK1 fragments are used, they preferably comprise at least 10, and more preferably, at least 20 contiguous amino acids of an GALK1 protein. GALK1-specific antigens and/or immunogens may be coupled to carrier proteins that stimulate the immune response. For example, the subject polypeptides are covalently coupled to the keyhole limpet hemocyanin (KLH) carrier, and the conjugate is emulsified in Freund's complete adjuvant, which enhances the immune response. An appropriate immune system such as a laboratory rabbit or mouse is immunized according to conventional protocols.

The presence of GALK1-specific antibodies is assayed by an appropriate assay such as a solid phase enzyme-linked immunosorbant assay (ELISA) using immobilized corresponding GALK1 polypeptides. Other assays, such as radioimmunoassays or fluorescent assays might also be used.

Chimeric antibodies specific to GALK1 polypeptides can be made that contain different portions from different animal species. For instance, a human immunoglobulin constant region may be linked to a variable region of a murine mAb, such that the antibody derives its biological activity from the human antibody, and its binding specificity from the murine fragment. Chimeric antibodies are produced by splicing together genes that encode the appropriate regions from each species (Morrison et al., Proc. Natl. Acad. Sci. (1984) 81:6851-6855; Neuberger et al., Nature (1984) 312:604-608; Takeda et al., Nature (1985) 31:452-454). Humanized antibodies, which are a form of chimeric antibodies, can be generated by grafting complementary-determining regions (CDRs) (Carlos, T. M., J. M. Harlan. 1994. Blood 84:2068-2101) of mouse antibodies into a background of human framework regions and constant regions by recombinant DNA technology (Riechmann LM, et al., 1988 Nature 323: 323-327). Humanized antibodies contain ∼10% murine sequences and ∼90% human sequences, and thus further reduce or eliminate immunogenicity, while retaining the antibody specificities (Co MS, and Queen C. 1991 Nature 351: 501-501; Morrison SL. 1992 Ann. Rev. Immun. 10:239-265). Humanized antibodies and methods of their production are well-known in the art (U.S. Pat. Nos. 5,530,101, 5,585,089, 5,693,762, and 6,180,370).

GALK1-specific single chain antibodies which are recombinant, single chain polypeptides formed by linking the heavy and light chain fragments of the Fv regions via an amino acid bridge, can be produced by methods known in the art (U.S. Pat. No. 4,946,778; Bird, Science (1988) 242:423-426; Huston et al., Proc. Natl. Acad. Sci. USA (1988) 85:5879-5883; and Ward et al., Nature (1989) 334:544-546).

Other suitable techniques for antibody production involve in vitro exposure of lymphocytes to the antigenic polypeptides or alternatively to selection of libraries of antibodies in phage or similar vectors (Huse et al., Science (1989) 246:1275-1281). As used herein, T-cell antigen receptors are included within the scope of antibody modulators (Harlow and Lane, 1988, *supra*).

The polypeptides and antibodies may be used with or without modification. Frequently, antibodies will be labeled by joining, either covalently or non-covalently, a substance that provides for a detectable signal, or that is toxic to cells that express the targeted protein (Menard S, et al., Int J. Biol Markers (1989) 4:131-134). A wide variety of labels and conjugation techniques are known and are reported extensively in both the scientific and patent literature. Suitable labels include radionuclides, enzymes, substrates, cofactors, inhibitors, fluorescent moieties, fluorescent emitting lanthanide metals, chemiluminescent moieties, bioluminescent moieties, magnetic particles, and the like (U.S. Pat. Nos. 3,817,837; 3,850,752; 3,939,350; 3,996,345; 4,277,437; 4,275,149; and 4,366,241). Also, recombinant immunoglobulins may be produced (U.S. Pat. No. 4,816,567). Antibodies to cytoplasmic polypeptides may be delivered and reach their targets by conjugation with membrane-penetrating toxin proteins (U.S. Pat. No. 6,086,900).

When used therapeutically in a patient, antibodies are typically administered parenterally, when possible at the target site, or intravenously. The therapeutically effective dose and dosage regimen is determined by clinical studies. Typically, the amount of antibody administered is in the range of about 0.1 mg/kg -to about 10 mg/kg of patient weight. For parenteral administration, the antibodies are formulated in a unit dosage injectable form (e.g., solution, suspension, emulsion) in association with a pharmaceutically acceptable vehicle. Such vehicles are inherently nontoxic and non-therapeutic. Examples are water, saline, Ringer's solution, dextrose solution, and 5% human serum albumin. Nonaqueous vehicles such as fixed oils, ethyl oleate, or liposome carriers may also be used. The vehicle may contain minor amounts of additives, such as buffers and preservatives, which enhance isotonicity and chemical stability or otherwise enhance therapeutic potential. The antibodies' concentrations in such vehicles are typically in the range of about 1 mg/ml to about10 mg/ml. Immunotherapeutic methods are further described in the literature (US Pat. No. 5,859,206; WO0073469).

### Specific biotherapeutics

An GALK1-interacting protein may have biotherapeutic applications. Biotherapeutic agents formulated in pharmaceutically acceptable carriers and dosages may be used to activate or inhibit signal transduction pathways. This modulation may be accomplished by binding a ligand, thus inhibiting the activity of the pathway; or by binding a receptor, either to inhibit activation of, or to activate, the receptor. Alternatively, the biotherapeutic may itself be a ligand capable of activating or inhibiting a receptor. Biotherapeutic agents and methods of producing them are described in detail in U.S. Pat. No. 6,146,628.

When the GALK1 is a ligand, it may be used as a biotherapeutic agent to activate or inhibit its natural receptor. Alternatively, antibodies against GALK1, as described in the previous section, may be used as biotherapeutic agents.

When the GALK1 is a receptor, its ligand(s), antibodies to the ligand(s) or the GALK1 itself may be used as biotherapeutics to modulate the activity of GALK1 in the PTEN/AKT pathway.

### Nucleic Acid Modulators

Other preferred GALK1-modulating agents comprise nucleic acid molecules, such as antisense oligomers or double stranded RNA (dsRNA), which generally inhibit GALK1 activity. Preferred nucleic acid modulators interfere with the function of the GALK1 nucleic acid such as DNA replication, transcription, translocation of the GALK1 RNA to the site of protein translation, translation of protein from the GALK1 RNA, splicing of the GALK1 RNA to yield one or more mRNA species, or catalytic activity which may be engaged in or facilitated by the GALK1 RNA.

In one embodiment, the antisense oligomer is an oligonucleotide that is sufficiently complementary to an GALK1 mRNA to bind to and prevent translation, preferably by binding to the 5' untranslated region. GALK1-specific antisense oligonucleotides, preferably range from at least 6 to about 200 nucleotides. In some embodiments the oligonucleotide is preferably at least 10, 15, or 20 nucleotides in length. In other embodiments, the oligonucleotide is preferably less than 50, 40, or 30 nucleotides in length. The oligonucleotide can be DNA or RNA or a chimeric mixture or derivatives or modified versions thereof, single-stranded or double-stranded. The oligonucleotide can be modified at the base moiety, sugar moiety, or phosphate backbone. The oligonucleotide may include other appending groups such as peptides, agents that facilitate transport across the cell membrane, hybridization-triggered cleavage agents, and intercalating agents.

In another embodiment, the antisense oligomer is a phosphothioate morpholino oligomer (PMO). PMOs are assembled from four different morpholino subunits, each of which contain one of four genetic bases (A, C, G, or T) linked to a six-membered morpholine ring. Polymers of these subunits are joined by non-ionic phosphodiamidate intersubunit linkages. Details of how to make and use PMOs and other antisense oligomers are well known in the art (e.g. see WO99/18193; Probst JC, Antisense Oligodeoxynucleotide and Ribozyme Design, Methods. (2000) 22(3):271-281; Summerton J, and Weller D. 1997 Antisense Nucleic Acid Drug Dev. :7:187-95; US Pat. No. 5,235,033; and US Pat No. 5,378,841).

Alternative preferred GALK1 nucleic acid modulators are double-stranded RNA species mediating RNA interference (RNAi). RNAi is the process of sequence-specific, post-transcriptional gene silencing in animals and plants, initiated by double-stranded RNA (dsRNA) that is homologous in sequence to the silenced gene. Methods relating to the use of RNAi to silence genes in *C*. *elegans, Drosophila,* plants, and humans are known in the art (Fire A, et al., 1998 Nature 391:806-811; Fire, A. Trends Genet. 15, 358-363 (1999); Sharp, P. A. RNA interference 2001. Genes Dev. 15,485-490 (2001); Hammond, S. M., et al., Nature Rev. Genet. 2, 110-1119 (2001); Tuschl, T. Chem. Biochem. 2,239-245 (2001); Hamilton, A. et al., Science 286, 950-952 (1999); Hammond, S. M., et al., Nature 404, 293-296 (2000); Zamore, P. D., et al., Cell 101, 25-33 (2000); Bernstein, E., et al., Nature 409, 363-366 (2001); Elbashir, S. M., et al., Genes Dev. 15, 188-200 (2001); WO0129058; WO9932619; Elbashir SM, et al., 2001 Nature 411:494-498; Novina CD and Sharp P. 2004 Nature 430:161-164; Soutschek J et al 2004 Nature 432:173-178; Hsieh AC et al. (2004) NAR 32(3):893-901).

Nucleic acid modulators are commonly used as research reagents, diagnostics, and therapeutics. For example, antisense oligonucleotides, which are able to inhibit gene expression with exquisite specificity, are often used to elucidate the function of particular genes (see, for example, U.S. Pat. No. 6,165,790). Nucleic acid modulators are also used, for example, to distinguish between functions of various members of a biological pathway. For example, antisense oligomers have been employed as therapeutic moieties in the treatment of disease states in animals and man and have been demonstrated in numerous clinical trials to be safe and effective (Milligan JF, et al, Current Concepts in Antisense Drug Design, J Med Chem. (1993) 36:1923-1937; Tonkinson JL et al., Antisense Oligodeoxynucleotides as Clinical Therapeutic Agents, Cancer Invest. (1996) 14:54-65). Accordingly, in one aspect of the invention, an GALK1-specific nucleic acid modulator is used in an assay to further elucidate the role of the GALK1 in the PTEN/AKT pathway, and/or its relationship to other members of the pathway. A GALK1-specific antisense oligomer may be used as a therapeutic agent for treatment of PTEN/AKT-related disease states.

### Assay Systems

The invention provides assay systems and screening methods for identifying specific modulators of GALK1 activity. As used herein, an "assay system" encompasses all the components required for performing and analyzing results of an assay that detects and/or measures a particular event. In general, primary assays are used to identify or confirm a modulator's specific biochemical or molecular effect with respect to the GALK1 nucleic acid or protein. In general, secondary assays further assess the activity of an GALK1 modulating agent identified by a primary assay and may confirm that the modulating agent affects GALK1 in a manner relevant to the PTEN/AKT pathway. In some cases, GALK1 modulators will be directly tested in a secondary assay.

In a preferred embodiment, the screening method comprises contacting a suitable assay system comprising an GALK1 polypeptide or nucleic acid with a candidate agent under conditions whereby, but for the presence of the agent, the system provides a reference activity (e.g. binding activity), which is based on the particular molecular event the screening method detects. A statistically significant difference between the agent-biased activity and the reference activity indicates that the candidate agent modulates GALK1 activity, and hence the PTEN/AKT pathway. The GALK1 polypeptide or nucleic acid used in the assay may comprise any of the nucleic acids or polypeptides described above.

### Primary Assays

The type of modulator tested generally determines the type of primary assay.

### Primary assays for small molecule modulators

For small molecule modulators, screening assays are used to identify candidate modulators. Screening assays may be cell-based or may use a cell-free system that recreates or retains the relevant biochemical reaction of the target protein (reviewed in Sittampalam GS et al., Curr Opin Chem Biol (1997) 1:384-91 and accompanying references). As used herein the term "cell-based" refers to assays using live cells, dead cells, or a particular cellular fraction, such as a membrane, endoplasmic reticulum, or mitochondrial fraction. The term "cell free" encompasses assays using substantially purified protein (either endogenous or recombinantly produced), partially purified or crude cellular extracts. Screening assays may detect a variety of molecular events, including protein-DNA interactions, protein-protein interactions (*e.g*., receptor-ligand binding), transcriptional activity (*e.g*., using a reporter gene), enzymatic activity (*e.g*., via a property of the substrate), activity of second messengers, immunogenicty and changes in cellular morphology or other cellular characteristics. Appropriate screening assays may use a wide range of detection methods including fluorescent, radioactive, colorimetric, spectrophotometric, and amperometric methods, to provide a read-out for the particular molecular event detected.

Cell-based screening assays usually require systems for recombinant expression of GALK1 and any auxiliary proteins demanded by the particular assay. Appropriate methods for generating recombinant proteins produce sufficient quantities of proteins that retain their relevant biological activities and are of sufficient purity to optimize activity and assure assay reproducibility. Yeast two-hybrid and variant screens, and mass spectrometry provide preferred methods for determining protein-protein interactions and elucidation of protein complexes. In certain applications, when GALK1-interacting proteins are used in screens to identify small molecule modulators, the binding specificity of the interacting protein to the GALK1 protein may be assayed by various known methods such as substrate processing (e.g. ability of the candidate GALK1-specific binding agents to function as negative effectors in GALK1-expressing cells), binding equilibrium constants (usually at least about 10⁷ M⁻¹, preferably at least about 10⁸ M⁻¹, more preferably at least about 10⁹ M⁻¹), and immunogenicity (e.g. ability to elicit GALK1 specific antibody in a heterologous host such as a mouse, rat, goat or rabbit). For enzymes and receptors, binding may be assayed by, respectively, substrate and ligand processing.

The screening assay may measure a candidate agent's ability to specifically bind to or modulate activity of an GALK1 polypeptide, a fusion protein thereof, or to cells or membranes bearing the polypeptide or fusion protein. The GALK1 polypeptide can be full length or a fragment thereof that retains functional GALK1 activity. The GALK1 polypeptide may be fused to another polypeptide, such as a peptide tag for detection or anchoring, or to another tag. The GALK1 polypeptide is preferably human GALK1, or is an ortholog or derivative thereof as described above. In a preferred embodiment, the screening assay detects candidate agent-based modulation of GALK1 interaction with a binding target, such as an endogenous or exogenous protein or other substrate that has GALK1 -specific binding activity, and can be used to assess normal GALK1 gene function.

Suitable assay formats that may be adapted to screen for GALK1 modulators are known in the art. Preferred screening assays are high throughput or ultra high throughput and thus provide automated, cost-effective means of screening compound libraries for lead compounds (Fernandes PB, Curr Opin Chem Biol (1998) 2:597-603; Sundberg SA, Curr Opin Biotechnol 2000, 11:47-53). In one preferred embodiment, screening assays uses fluorescence technologies, including fluorescence polarization, time-resolved fluorescence, and fluorescence resonance energy transfer. These systems offer means to monitor protein-protein or DNA-protein interactions in which the intensity of the signal emitted from dye-labeled molecules depends upon their interactions with partner molecules (*e.g*., Selvin PR, Nat Struct Biol (2000) 7:730-4; Fernandes PB, *supra*; Hertzberg RP and Pope AJ, Curr Opin Chem Biol (2000) 4:445-451).

A variety of suitable assay systems may be used to identify candidate GALK1 and PTEN/AKT pathway modulators (e.g. U.S. Pat. No. 6,165,992 and U.S. Pat. No. 6720162 (kinase assays); U.S. Pat. Nos. 5,550,019 and 6,133,437 (apoptosis assays); U.S. Pat. No. 6,114,132 and U.S. Pat. No. 6720162 (phosphatase and protease assays); and U.S. Pat. Nos. 5,976,782, 6,225,118 and 6,444,434 (angiogenesis assays), among others). Specific preferred assays are described in more detail below.

Protein kinases, key signal transduction proteins that may be either membrane-associated or intracellular, catalyze the transfer of gamma phosphate from adenosine triphosphate (ATP) to a serine, threonine or tyrosine residue in a protein substrate. Radioassays, which monitor the transfer from [gamma-³²P or -³³P]ATP, are frequently used to assay kinase activity. For instance, a scintillation assay for p56 (lck) kinase activity monitors the transfer of the gamma phosphate from [gamma -³³P] ATP to a biotinylated peptide substrate. The substrate is captured on a streptavidin coated bead that transmits the signal (Beveridge M et al., J Biomol Screen (2000) 5:205-212). This assay uses the scintillation proximity assay (SPA), in which only radio-ligand bound to receptors tethered to the surface of an SPA bead are detected by the scintillant immobilized within it, allowing binding to be measured without separation of bound from free ligand. Other assays for protein kinase activity may use antibodies that specifically recognize phosphorylated substrates. For instance, the kinase receptor activation (KIRA) assay measures receptor tyrosine kinase activity by ligand stimulating the intact receptor in cultured cells, then capturing solubilized receptor with specific antibodies and quantifying phosphorylation via phosphotyrosine ELISA (Sadick MD, Dev Biol Stand (1999) 97:121-133). Another example of antibody based assays for protein kinase activity is TRF (time-resolved fluorometry). This method utilizes europium chelate-labeled anti-phosphotyrosine antibodies to detect phosphate transfer to a polymeric substrate coated onto microtiter plate wells. The amount of phosphorylation is then detected using time-resolved, dissociation-enhanced fluorescence (Braunwalder AF, et al., Anal Biochem 1996 Jul 1;238(2):159-64). Yet other assays for kinases involve uncoupled, pH sensitive assays that can be used for high-throughput screening of potential inhibitors or for determining substrate specificity. Since kinases catalyze the transfer of a gamma-phosphoryl group from ATP to an appropriate hydroxyl acceptor with the release of a proton, a pH sensitive assay is based on the detection of this proton using an appropriately matched buffer/indicator system (Chapman E and Wong CH (2002) Bioorg Med Chem. 10:551-5).

Protein phosophatases catalyze the removal of a gamma phosphate from a serine, threonine or tyrosine residue in a protein substrate. Since phosphatases act in opposition to kinases, appropriate assays measure the same parameters as kinase assays. In one example, the dephosphorylation of a fluorescently labeled peptide substrate allows trypsin cleavage of the substrate, which in turn renders the cleaved substrate significantly more fluorescent (Nishikata M et al., Biochem J (1999) 343:35-391). In another example, fluorescence polarization (FP), a solution-based, homogeneous technique requiring no immobilization or separation of reaction components, is used to develop high throughput screening (HTS) assays for protein phosphatases. This assay uses direct binding of the phosphatase with the target, and increasing concentrations of target- phosphatase increase the rate of dephosphorylation, leading to a change in polarization (Parker GJ et al., (2000) J Biomol Screen 5:77-88).

Proteases are enzymes that cleave protein substrates at specific sites. Exemplary assays detect the alterations in the spectral properties of an artificial substrate that occur upon protease-mediated cleavage. In one example, synthetic caspase substrates containing four amino acid proteolysis recognition sequences, separating two different fluorescent tags are employed; fluorescence resonance energy transfer detects the proximity of these fluorophores, which indicates whether the substrate is cleaved (Mahajan NP et al., Chem Biol (1999) 6:401-409).

Endogenous protease inhibitors may inhibit protease activity. In an example of an assay developed for either proteases or protease inhibitors, a biotinylated substrate is coated on a titer plate and hydrolyzed with the protease; the unhydrolyzed substrate is quantified by reaction with alkaline phosphatase-streptavidin complex and detection of the reaction product. The activity of protease inhibitors correlates with the activity of the alkaline phosophatase indicator enzyme (Gan Z et al., Anal Biochem 1999) 268:151-156).

Glycosyltransferases mediate changes in glycosylation patterns that, in turn, may affect the function of glycoproteins and/or glycolipids and, further downstream, processes of development, differentiation, transformation and cell-cell recognition. An assay for glycosyltransferase uses scintillation methods to measure the transfer of carbohydrate from radiolabeled sugar-nuecleotide donor to a synthetic glycopolymer acceptor that is coupled to polyacrylamide and coated on plastic microtiter plates (Donovan RS et al., Glycoconj J (1999) 16:607-615).

Histone deacetylation and acetylation proteins are involved in regulating chromatin structure during transcription and thus function in gene regulation. In one example, a histone deacetylase assay uses the scintillation proximity assay (SPA) and biotinylated [3H]acetyl histone H4 peptide substrate (Nare B et al., Anal Biochem 1999, 267:390-396). Upon binding to streptavidin-coated SPA beads, the peptide substrate generates a radioactive signal, which decreases as a result of histone deacetylase activity.

G-protein-coupled receptors (GPCRs) comprise a large family of cell surface receptors that mediate a diverse array of biological functions. They selectively respond to a wide variety of extracellular chemical stimuli to activate specific signaling cascades. Assays may measure reporter gene activity or changes in intracellular calcium ions, or other second messengers (Durocher Y et al., Anal Biochem (2000) 284: 316-326; Miller TR et al., J Biomol Screen (1999) 4:249-258). Such assays may utilize chimeric Ga proteins that will couple to many different GPCRs and thus facilitate "universal" screening assays (Coward P et al., Anal Biochem (1999) 270:242-248; Milligan G and Rees S et al., Trends Pharmacol Sci (1999) 20:118-124).

GPCRs exert their effects through heterotrimeric G proteins, which cycle between active GTP- and inactive GDP-bound forms. Receptors catalyze the activation of G proteins by promoting exchange of GDP for GTP, while G proteins catalyze their own deactivation through their intrinsic GTPase activity. GEFs accelerate GDP dissociation and GTP binding, while GAPs stimulate GTP hydrolysis to GDP. The same assays used to monitor GPCR activity may thus be applied to monitor the activity of GEFs or GAPs. Alternatively, GEF activity may be assayed by the release of labeled GDP from the appropriate GTPase or by the uptake of labelled GTP. GAP activity may be monitored via a GTP hydrolysis assay using labeled GTP (*e.g*., Jones S et al., Molec Biol Cell (1998) 9:2819-2837).

Transporter proteins carry a range of substrates, including nutrients, ions, amino acids, and drugs, across cell membranes. Assays for modulators of transporters may use labeled substrates. For instance, exemplary high throughput screens to identify compounds that interact with different peptide and anion transporters both use fluorescently labeled substrates; the assay for peptide transport additionally uses multiscreen filtration plates (Blevitt JM et al., J Biomol Screen 1999, 4:87-91; Cihlar T and Ho ES, Anal Biochem 2000,283:49-55).

Ion channels mediate essential physiological functions, including fluid secretion, electrolyte balance, bioenergetics, and membrane excitability. Assays for channel activity can incorporate ion-sensitive dyes or proteins or voltage-sensitive dyes or proteins, as reviewed in Gonzalez JE et al. (Drug Discovery Today (1999) 4:431-439). Alternative methods measure the displacement of known ligands, which may be radio-labeled or fluorescently labeled (*e.g*., Schmid EL et al., Anal Chem (1998) 70:1331-1338).

Reductases are enzymes of oxidoreductase class that catalyze reactions in which metabolites are reduced. High throughput screening assays for reductases may involve scintillation (Fernandes PB. (1998) Curr Opin Chem Biol 2:597-603; Delaporte E et al. (2001) J Biomol Screen 6:225-231).

Hydrolases catalyze the hydrolysis of a substrate such as esterases, lipases, peptidases, nucleotidases, and phosphatases, among others. Enzyme activity assays may be used to measure hydrolase activity. The activity of the enzyme is determined in presence of excess substrate, by spectrophotometrically measuring the rate of appearance of reaction products. High throughput arrays and assays for hydrolases are known to those skilled in the art (Park CB and Clark DS (2002) Biotech Bioeng 78:229-235).

Kinesins are motor proteins. Assays for kinesins involve their ATPase activity, such as described in Blackburn et al (Blackburn CL, et al., (1999) J Org Chem 64:5565-5570). The ATPase assay is performed using the EnzCheck ATPase kit (Molecular Probes). The assays are performed using an Ultraspec spectrophotometer (Pharmacia), and the progress of the reaction are monitored by absorbance increase at 360 nm. Microtubules (1.7 mM final), kinesin (0.11 mM final), inhibitor (or DMSO blank at 5% final), and the EnzCheck components (purine nucleotide phosphorylase and MESG substrate) are premixed in the cuvette in a reaction buffer (40 mM PIPES pH 6.8, 5 mM paclitaxel, 1 mM EGTA, 5 mM MgCl2). The reaction is initiated by addition of MgATP (1 mM final).

Peptidyl-prolyl isomerase (PPIase) proteins, which include cyclophilins, FK506 binding proteins and paravulins, catalyze the isomerization of *cis-trans* proline peptide bonds in oligopeptides and are thought to be essential for protein folding during protein synthesis in the cell. Spectrophotometric assays for PPIase activity can detect isomerization of labeled peptide substrates, either by direct measurement of isomer-specific absorbance, or by coupling isomerization to isomer-specific cleavage by chymotrypsin (Scholz C et al., FEBS Lett (1997) 414:69-73; Janowski B et al., Anal Biochem (1997) 252:299-307; Kullertz G et al., Clin Chem (1998) 44:502-8). Alternative assays use the scintillation proximity or fluorescence polarization assay to screen for ligands of specific PPIases (Graziani F et al., J Biolmol Screen (1999) 4:3-7; Dubowchik GM et al., Bioorg Med Chem Lett (2000) 10:559-562). Assays for 3,2-trans-enoyl-CoA isomerase activity have also been described (Binstock, J. F., and Schulz, H. (1981) Methods Enzymol. 71:403-411; Geisbrecht BV et al (1999) J Biol Chem. 274:21797-803). These assays use 3-cis-octenoyl-CoA as a substrate, and reaction progress is monitored spectrophotometrically using a coupled assay for the isomerization of 3-cis-octenoyl-CoA to 2-trans-octenoyl-CoA. Assays for 3,2-trans-enoyl-CoA isomerase activity have also been described (Binstock, J. F., and Schulz, H. (1981) Methods Enzymol. 71:403-411; Geisbrecht BV et al (1999) J Biol Chem. 274:21797-803). These assays use 3-cis-octenoyl-CoA as a substrate, and reaction progress is monitored spectrophotometrically using a coupled assay for the isomerization of 3-cis-octenoyl-CoA to 2-trans-octenoyl-CoA.

High-throughput assays, such as scintillation proximity assays, for synthase enzymes involved in fatty acid synthesis are known in the art (He X et al (2000) Anal Biochem 2000 Jun 15;282(1):107-14).

**Apoptosis assays.** Apoptosis or programmed cell death is a suicide program is activated within the cell, leading to fragmentation of DNA, shrinkage of the cytoplasm, membrane changes and cell death. Apoptosis is mediated by proteolytic enzymes of the caspase family. Many of the altering parameters of a cell are measurable during apoptosis. Assays for apoptosis may be performed by terminal deoxynucleotidyl transferase-mediated digoxigenin-11-DUTP nick end labeling (TUNEL) assay. The TUNEL assay is used to measure nuclear DNA fragmentation characteristic of apoptosis ( Lazebnik et al., 1994, Nature 371, 346), by following the incorporation of fluorescein-dUTP (Yonehara et al., 1989, J. Exp. Med. 169, 1747). Apoptosis may further be assayed by acridine orange staining of tissue culture cells (Lucas, R., et al., 1998, Blood 15:4730-41). Other cell-based apoptosis assays include the caspase-3/7 assay and the cell death nucleosome ELISA assay. The caspase 3/7 assay is based on the activation of the caspase cleavage activity as part of a cascade of events that occur during programmed cell death in many apoptotic pathways. In the caspase 3/7 assay (commercially available Apo-ONE^{™} Homogeneous Caspase-3/7 assay from Promega, cat# 67790), lysis buffer and caspase substrate are mixed and added to cells. The caspase substrate becomes fluorescent when cleaved by active caspase 3/7. The nucleosome ELISA assay is a general cell death assay known to those skilled in the art, and available commercially (Roche, Cat# 1774425). This assay is a quantitative sandwich-enzyme-immunoassay which uses monoclonal antibodies directed against DNA and histones respectively, thus specifically determining amount of mono- and oligonucleosomes in the cytoplasmic fraction of cell lysates. Mono and oligonucleosomes are enriched in the cytoplasm during apoptosis due to the fact that DNA fragmentation occurs several hours before the plasma membrane breaks down, allowing for accumalation in the cytoplasm. Nucleosomes are not present in the cytoplasmic fraction of cells that are not undergoing apoptosis. The Phospho-histone H2B assay is another apoptosis assay, based on phosphorylation of histone H2B as a result of apoptosis. Fluorescent dyes that are associated with phosphohistone H2B may be used to measure the increase of phosphohistone H2B as a result of apoptosis. Apoptosis assays that simultaneously measure multiple parameters associated with apoptosis have also been developed. In such assays, various cellular parameters that can be associated with antibodies or fluorescent dyes, and that mark various stages of apoptosis are labeled, and the results are measured using instruments such as Cellomics™ ArrayScan^{®} HCS System. The measurable parameters and their markers include anti-active caspase-3 antibody which marks intermediate stage apoptosis, anti-PARP-p85 antibody (cleaved PART) which marks late stage apoptosis, Hoechst labels which label the nucleus and are used to measure nuclear swelling as a measure of early apoptosis and nuclear condensation as a measure of late apoptosis, TOTO-3 fluorescent dye which labels DNA of dead cells with high cell membrane permeability, and anti-alpha-tubulin or F-actin labels, which assess cytoskeletal changes in cells and correlate well with TOTO-3 label..

An apoptosis assay system may comprise a cell that expresses an GALK1, and that optionally has defective PTEN and/or AKT function (e.g. PTEN and/or AKT is over-expressed or under-expressed relative to wild-type cells). A test agent can be added to the apoptosis assay system and changes in induction of apoptosis relative to controls where no test agent is added, identify candidate PTEN/AKT modulating agents. In some embodiments of the invention, an apoptosis assay may be used as a secondary assay to test a candidate PTEN/AKT modulating agents that is initially identified using a cell-free assay system. An apoptosis assay may also be used to test whether GALK1 function plays a direct role in apoptosis. For example, an apoptosis assay may be performed on cells that over- or under-express GALK1 relative to wild type cells. Differences in apoptotic response compared to wild type cells suggests that the GALK1 plays a direct role in the apoptotic response. Apoptosis assays are described further in US Pat. No. 6,133,437.

**Cell proliferation and cell cycle assays**. Cell proliferation may be assayed via bromodeoxyuridine (BRDU) incorporation. This assay identifies a cell population undergoing DNA synthesis by incorporation of BRDU into newly-synthesized DNA. Newly-synthesized DNA may then be detected using an anti-BRDU antibody (Hoshino et al., 1986, Int. J. Cancer 38, 369; Campana et al., 1988, J. Immunol. Meth. 107, 79), or by other means.

Cell proliferation is also assayed via phospho-histone H3 staining, which identifies a cell population undergoing mitosis by phosphorylation of histone H3. Phosphorylation of histone H3 at serine 10 is detected using an antibody specfic to the phosphorylated form of the serine 10 residue of histone H3. (Chadlee,D.N. 1995, J. Biol. Chem 270:20098-105). Cell Proliferation may also be examined using [³H]-thymidine incorporation (Chen, J., 1996, Oncogene 13:1395-403; Jeoung, J., 1995, J. Biol. Chem. 270:18367-73). This assay allows for quantitative characterization of S-phase DNA syntheses. In this assay, cells synthesizing DNA will incorporate [³H]-thymidine into newly synthesized DNA. Incorporation can then be measured by standard techniques such as by counting of radioisotope in a scintillation counter (e.g., Beckman LS 3800 Liquid Scintillation Counter). Another proliferation assay uses the dye Alamar Blue (available from Biosource International), which fluoresces when reduced in living cells and provides an indirect measurement of cell number (Voytik-Harbin SL et al., 1998, In Vitro Cell Dev Biol Anim 34:239-46). Yet another proliferation assay, the MTS assay, is based on in vitro cytotoxicity assessment of industrial chemicals, and uses the soluble tetrazolium salt, MTS. MTS assays are commercially available, for example, the Promega CellTiter 96^{®} AQueous NonRadioactive Cell Proliferation Assay (Cat.# G5421).

Cell proliferation may also be assayed by colony formation in soft agar, or clonogenic survival assay (Sambrook et al., Molecular Cloning, Cold Spring Harbor (1989)). For example, cells transformed with GALK1 are seeded in soft agar plates, and colonies are measured and counted after two weeks incubation.

Cell proliferation may also be assayed by measuring ATP levels as indicator of metabolically active cells. Such assays are commercially available, for example Cell Titer-Glo™, which is a luminescent homogeneous assay available from Promega.

Involvement of a gene in the cell cycle may be assayed by flow cytometry (Gray JW et al. (1986) Int J Radiat Biol Relat Stud Phys Chem Med 49:237-55). Cells transfected with an GALK1 may be stained with propidium iodide and evaluated in a flow cytometer (available from Becton Dickinson), which indicates accumulation of cells in different stages of the cell cycle.

Involvement of a gene in cell cycle may also be assayed by FOXO nuclear translocation assays. The FOXO family of transcription factors are mediators of various cellular functions including cell cycle progression and cell death, and are negatively regulated by activation of the PI3 kinase pathway. Akt phosphorylation of FOXO family members leads to FOXO sequestration in the cytoplasm and transcriptional inactivation (Medema, R. H et al (2000) Nature 404: 782-787). PTEN is a negative regulator of PI3 kinase pathway. Activation of PTEN, or loss of PI3 kinase or AKT, prevents phosphorylation of FOXO, leading to accumulation of FOXO in the nucleus, transcriptional activation of FOXO regulated genes, and apoptosis. Alternatively, loss of PTEN leads to pathway activation and cell survival (Nakamura, N. et al (2000) Mol Cell Biol 20: 8969-8982). FOXO translocation into the cytoplasm is used in assays and screens to identify members and/or modulators of the PTEN pathway. FOXO translocation assays using GFP or luciferase as detection reagents are known in the art (e.g., Zhang X et al (2002) J Biol Chem 277:45276-45284; and Li et al (2003) Mol Cell Biol 23:104-118).

Accordingly, a cell proliferation or cell cycle assay system may comprise a cell that expresses an GALK1, and that optionally has defective PTEN/AKT function (e.g. PTEN and/or AKT is over-expressed or under-expressed relative to wild-type cells). A test agent can be added to the assay system and changes in cell proliferation or cell cycle relative to controls where no test agent is added, identify candidate PTEN/AKT modulating agents. In some embodiments of the invention, the cell proliferation or cell cycle assay may be used as a secondary assay to test a candidate PTEN/AKT modulating agents that is initially identified using another assay system such as a cell-free assay system. A cell proliferation assay may also be used to test whether GALK1 function plays a direct role in cell proliferation or cell cycle. For example, a cell proliferation or cell cycle assay may be performed on cells that over- or under-express GALK1 relative to wild type cells. Differences in proliferation or cell cycle compared to wild type cells suggests that the GALK1 plays a direct role in cell proliferation or cell cycle.

**Angiogenesis.** Angiogenesis may be assayed using various human endothelial cell systems, such as umbilical vein, coronary artery, or dermal cells. Suitable assays include Alamar Blue based assays (available from Biosource International) to measure proliferation; migration assays using fluorescent molecules, such as the use of Becton Dickinson Falcon HTS FluoroBlock cell culture inserts to measure migration of cells through membranes in presence or absence of angiogenesis enhancer or suppressors; and tubule formation assays based on the formation of tubular structures by endothelial cells on Matrigel® (Becton Dickinson). Accordingly, an angiogenesis assay system may comprise a cell that expresses an GALK1, and that optionally has defective PTEN and/or AKT function (e.g. PTEN and/or AKT is over-expressed or under-expressed relative to wild-type cells). A test agent can be added to the angiogenesis assay system and changes in angiogenesis relative to controls where no test agent is added, identify candidate PTEN/AKT modulating agents. In some embodiments of the invention, the angiogenesis assay may be used as a secondary assay to test a candidate PTEN/AKT modulating agents that is initially identified using another assay system. An angiogenesis assay may also be used to test whether GALK1 function plays a direct role in cell proliferation. For example, an angiogenesis assay may be performed on cells that over- or under-express GALK1 relative to wild type cells. Differences in angiogenesis compared to wild type cells suggests that the GALK1 plays a direct role in angiogenesis. U.S. Pat. Nos. 5,976,782, 6,225,118 and 6,444,434, among others, describe various angiogenesis assays.

**Hypoxic induction.** The alpha subunit of the transcription factor, hypoxia inducible factor-1 (HIF-1), is upregulated in tumor cells following exposure to hypoxia in vitro. Under hypoxic conditions, HIF-1 stimulates the expression of genes known to be important in tumour cell survival, such as those encoding glyolytic enzymes and VEGF. Induction of such genes by hypoxic conditions may be assayed by growing cells transfected with GALK1 in hypoxic conditions (such as with 0.1% 02, 5% CO2, and balance N2, generated in a Napco 7001 incubator (Precision Scientific)) and normoxic conditions, followed by assessment of gene activity or expression by Taqman®. For example, a hypoxic induction assay system may comprise a cell that expresses an GALK1, and that optionally has defective PTEN and/or AKT function (e.g. PTEN and/or AKT is over-expressed or under-expressed relative to wild-type cells). A test agent can be added to the hypoxic induction assay system and changes in hypoxic response relative to controls where no test agent is added, identify candidate PTEN/AKT modulating agents. In some embodiments of the invention, the hypoxic induction assay may be used as a secondary assay to test a candidate PTEN/AKT modulating agents that is initially identified using another assay system. A hypoxic induction assay may also be used to test whether GALK1 function plays a direct role in the hypoxic response. For example, a hypoxic induction assay may be performed on cells that over- or under-express GALK1 relative to wild type cells. Differences in hypoxic response compared to wild type cells suggests that the GALK1 plays a direct role in hypoxic induction.

**Cell adhesion.** Cell adhesion assays measure adhesion of cells to purified adhesion proteins, or adhesion of cells to each other, in presence or absence of candidate modulating agents. Cell-protein adhesion assays measure the ability of agents to modulate the adhesion of cells to purified proteins. For example, recombinant proteins are produced, diluted to 2.5g/mL in PBS, and used to coat the wells of a microtiter plate. The wells used for negative control are not coated. Coated wells are then washed, blocked with 1% BSA, and washed again. Compounds are diluted to 2× final test concentration and added to the blocked, coated wells. Cells are then added to the wells, and the unbound cells are washed off. Retained cells are labeled directly on the plate by adding a membrane-permeable fluorescent dye, such as calcein-AM, and the signal is quantified in a fluorescent microplate reader.

Cell-cell adhesion assays measure the ability of agents to modulate binding of cell adhesion proteins with their native ligands. These assays use cells that naturally or recombinantly express the adhesion protein of choice. In an exemplary assay, cells expressing the cell adhesion protein are plated in wells of a multiwell plate. Cells expressing the ligand are labeled with a membrane-permeable fluorescent dye, such as BCECF , and allowed to adhere to the monolayers in the presence of candidate agents. Unbound cells are washed off, and bound cells are detected using a fluorescence plate reader.

High-throughput cell adhesion assays have also been described. In one such assay, small molecule ligands and peptides are bound to the surface of microscope slides using a microarray spotter, intact cells are then contacted with the slides, and unbound cells are washed off. In this assay, not only the binding specificity of the peptides and modulators against cell lines are determined, but also the functional cell signaling of attached cells using immunofluorescence techniques in situ on the microchip is measured (Falsey JR et al., Bioconjug Chem. 2001 May-Jun;12(3):346-53).

### Primary assays for antibody modulators

For antibody modulators, appropriate primary assays test is a binding assay that tests the antibody's affinity to and specificity for the GALK1 protein. Methods for testing antibody affinity and specificity are well known in the art (Harlow and Lane, 1988, 1999, *supra*). The enzyme-linked immunosorbant assay (ELISA) is a preferred method for detecting GALK1-specific antibodies; others include FACS assays, radioimmunoassays, and fluorescent assays.

In some cases, screening assays described for small molecule modulators may also be used to test antibody modulators.

### Primary assays for nucleic acid modulators

For nucleic acid modulators, primary assays may test the ability of the nucleic acid modulator to inhibit or enhance GALK1 gene expression, preferably mRNA expression. In general, expression analysis comprises comparing GALK1 expression in like populations of cells (*e.g*., two pools of cells that endogenously or recombinantly express GALK1) in the presence and absence of the nucleic acid modulator. Methods for analyzing mRNA and protein expression are well known in the art. For instance, Northern blotting, slot blotting, ribonuclease protection, quantitative RT-PCR (*e.g*., using the TaqMan®, PE Applied Biosystems), or microarray analysis may be used to confirm that GALK1 mRNA expression is reduced in cells treated with the nucleic acid modulator (*e.g*., Current Protocols in Molecular Biology (1994) Ausubel FM et al., eds., John Wiley & Sons, Inc., chapter 4; Freeman WM et al., Biotechniques (1999) 26:112-125; Kallioniemi OP, Ann Med 2001, 33:142-147; Blohm DH and Guiseppi-Elie, A Curr Opin Biotechnol 2001, 12:41-47). Protein expression may also be monitored. Proteins are most commonly detected with specific antibodies or antisera directed against either the GALK1 protein or specific peptides. A variety of means including Western blotting, ELISA, or in situ detection, are available (Harlow E and Lane D, 1988 and 1999, *supra*).

In some cases, screening assays described for small molecule modulators, particularly in assay systems that involve GALK1 mRNA expression, may also be used to test nucleic acid modulators.

### Secondary Assays

Secondary assays may be used to further assess the activity of GALK1-modulating agent identified by any of the above methods to confirm that the modulating agent affects GALK1 in a manner relevant to the PTEN/AKT pathway. As used herein, GALK1-modulating agents encompass candidate clinical compounds or other agents derived from previously identified modulating agent. Secondary assays can also be used to test the activity of a modulating agent on a particular genetic or biochemical pathway or to test the specificity of the modulating agent's interaction with GALK1.

Secondary assays generally compare like populations of cells or animals (*e.g*., two pools of cells or animals that endogenously or recombinantly express GALK1) in the presence and absence of the candidate modulator. In general, such assays test whether treatment of cells or animals with a candidate GALK1-modulating agent results in changes in the PTEN/AKT pathway in comparison to untreated (or mock- or placebo-treated) cells or animals. Certain assays use "sensitized genetic backgrounds", which, as used herein, describe cells or animals engineered for altered expression of genes in the PTEN/AKT or interacting pathways.

### Cell-based assays

Cell based assays may detect endogenous PTEN/AKT pathway activity or may rely on recombinant expression of PTEN/AKT pathway components. Any of the aforementioned assays may be used in this cell-based format. Candidate modulators are typically added to the cell media but may also be injected into cells or delivered by any other efficacious means.

### Animal Assays (not claimed)

A variety of non-human animal models of normal or defective PTEN/AKT pathway may be used to test candidate GALK1 modulators. Models for defective PTEN/AKT pathway typically use genetically modified animals that have been engineered to mis-express (*e.g*., over-express or lack expression in) genes involved in the PTEN/AKT pathway. Assays generally require systemic delivery of the candidate modulators, such as by oral administration, injection, etc.

PTEN/AKT pathway activity may be assessed by monitoring neovascularization and angiogenesis. Animal models with defective and normal PTEN/AKT may be used to test the candidate modulator's affect on GALK1 in Matrigel® assays. Matrigel® is an extract of basement membrane proteins, and is composed primarily of laminin, collagen IV, and heparin sulfate proteoglycan. It is provided as a sterile liquid at 4° C, but rapidly forms a solid gel at 37° C. Liquid Matrigel® is mixed with various angiogenic agents, such as bFGF and VEGF, or with human tumor cells which over-express the GALK1. The mixture is then injected subcutaneously(SC) into female athymic nude mice (Taconic, Germantown, NY) to support an intense vascular response. Mice with Matrigel® pellets may be dosed via oral (PO), intraperitoneal (IP), or intravenous (IV) routes with the candidate modulator. Mice are euthanized 5 - 12 days post-injection, and the Matrigel® pellet is harvested for hemoglobin analysis (Sigma plasma hemoglobin kit). Hemoglobin content of the gel is found to correlate the degree of neovascularization in the gel.

The effect of the candidate modulator on GALK1 may be assessed via tumorigenicity assays. Tumor xenograft assays are known in the art (see, e.g., Ogawa K et al., 2000, Oncogene 19:6043-6052). Xenografts are typically implanted SC into female athymic mice, 6-7 week old, as single cell suspensions either from a pre-existing tumor or from *in vitro* culture. The tumors which express the GALK1 endogenously are injected in the flank, 1 x 10⁵ to 1 x 10⁷ cells per mouse in a volume of 100 µL using a 27gauge needle. Mice are then ear tagged and tumors are measured twice weekly. Candidate modulator treatment is initiated on the day the mean tumor weight reaches 100 mg. Candidate modulator is delivered IV, SC, IP, or PO by bolus administration. Depending upon the pharmacokinetics of each unique candidate modulator, dosing can be performed multiple times per day. The tumor weight is assessed by measuring perpendicular diameters with a caliper and calculated by multiplying the measurements of diameters in two dimensions. At the end of the experiment, the excised tumors maybe utilized for biomarker identification or further analyses. For immunohistochemistry staining, xenograft tumors are fixed in 4% paraformaldehyde, 0.1M phosphate, pH 7.2, for 6 hours at 4°C, immersed in 30% sucrose in PBS, and rapidly frozen in isopentane cooled with liquid nitrogen.

Tumorogenicity may be monitored using a hollow fiber assay, which is described in U.S. Pat No. US 5,698,413. Briefly, the method comprises implanting into a laboratory animal a biocompatible, semi-permeable encapsulation device containing target cells, treating the laboratory animal with a candidate modulating agent, and evaluating the target cells for reaction to the candidate modulator. Implanted cells are generally human cells from a pre-existing tumor or a tumor cell line. After an appropriate period of time, generally around six days, the implanted samples are harvested for evaluation of the candidate modulator. Tumorogenicity and modulator efficacy may be evaluated by assaying the quantity of viable cells present in the macrocapsule, which can be determined by tests known in the art, for example, MTT dye conversion assay, neutral red dye uptake, trypan blue staining, viable cell counts, the number of colonies formed in soft agar, the capacity of the cells to recover and replicate in vitro, etc.

A tumorogenicity assay may use a transgenic animal, usually a mouse, carrying a dominant oncogene or tumor suppressor gene knockout under the control of tissue specific regulatory sequences; these assays are generally referred to as transgenic tumor assays. In one application, tumor development in the transgenic model is well characterized or is controlled. In an exemplary model, the "RIP1-Tag2" transgene, comprising the SV40 large T-antigen oncogene under control of the insulin gene regulatory regions is expressed in pancreatic beta cells and results in islet cell carcinomas (Hanahan D, 1985, Nature 315:115-122; Parangi S et al, 1996, Proc Natl Acad Sci USA 93: 2002-2007; Bergers G et al, 1999, Science 284:808-812). An "angiogenic switch," occurs at approximately five weeks, as normally quiescent capillaries in a subset of hyperproliferative islets become angiogenic. The RIP1-TAG2 mice die by age 14 weeks. Candidate modulators may be administered at a variety of stages, including just prior to the angiogenic switch (e.g., for a model of tumor prevention), during the growth of small tumors (e.g., for a model of intervention), or during the growth of large and/or invasive tumors (e.g., for a model of regression). Tumorogenicity and modulator efficacy can be evaluating life-span extension and/or tumor characteristics, including number of tumors, tumor size, tumor morphology, vessel density, apoptotic index, etc.

### Diagnostic and therapeutic uses (not claimed)

Specific GALK1-modulating agents are useful in a variety of diagnostic and therapeutic applications where disease or disease prognosis is related to defects in the PTEN/AKT pathway, such as angiogenic, apoptotic, or cell proliferation disorders. A method for modulating the PTEN/AKT pathway in a cell, for example a cell pre-determined to have defective or impaired PTEN/AKT function (e.g. due to overexpression, underexpression, or misexpression of PTEN/AKT, or due to gene mutations), may comprise the step of administering an agent to the cell that specifically modulates GALK1 activity. The modulating agent may produce a detectable phenotypic change in the cell indicating that the PTEN/AKT function is restored. The phrase "function is restored", and equivalents, as used herein, means that the desired phenotype is achieved, or is brought closer to normal compared to untreated cells. For example, with restored PTEN/AKT function, cell proliferation and/or progression through cell cycle may normalize, or be brought closer to normal relative to untreated cells. Methods for treating disorders or disease associated with impaired PTEN/AKT function may involve administering a therapeutically effective amount of an GALK1 -modulating agent that modulates the PTEN/AKT pathway. Methods for modulating GALK1 function in a cell, for example a cell pre-determined to have defective or impaired GALK1 function may involve administering an GALK1 -modulating agent. Additionally, a method for treating disorders or disease associated with impaired GALK1 function may involve administering a therapeutically effective amount of an GALK1 -modulating agent.

The discovery that GALK1 is implicated in PTEN/AKT pathway enables a variety of methods that can be employed for the diagnostic and prognostic evaluation of diseases and disorders involving defects in the PTEN/AKT pathway and for the identification of subjects having a predisposition to such diseases and disorders.

Various expression analysis methods can be used to diagnose whether GALK1 expression occurs in a particular sample, including Northern blotting, slot blotting, ribonuclease protection, quantitative RT-PCR, and microarray analysis. (*e.g*., Current Protocols in Molecular Biology (1994) Ausubel FM et al., eds., John Wiley & Sons, Inc., chapter 4; Freeman WM et al., Biotechniques (1999) 26:112-125; Kallioniemi OP, Ann Med 2001, 33:142-147; Blohm and Guiseppi-Elie, Curr Opin Biotechnol 2001, 12:41-47). Tissues having a disease or disorder implicating defective PTEN/AKT signaling that express an GALK1, are identified as amenable to treatment with an GALK1 modulating agent. In one application, the PTEN/AKT defective tissue overexpresses an GALK1 relative to normal tissue. For example, a Northern blot analysis of mRNA from tumor and normal cell lines, or from tumor and matching normal tissue samples from the same patient, using full or partial GALK1 cDNA sequences as probes, can determine whether particular tumors express or overexpress GALK1. Alternatively, the TaqMan® is used for quantitative RT-PCR analysis of GALK1 expression in cell lines, normal tissues and tumor samples (PE Applied Biosystems).

Various other diagnostic methods may be performed, for example, utilizing reagents such as the GALK1 oligonucleotides, and antibodies directed against an GALK1, as described above for: (1) the detection of the presence of GALK1 gene mutations, or the detection of either over- or under-expression of GALK1 mRNA relative to the non-disorder state; (2) the detection of either an over- or an under- abundance of GALK1 gene product relative to the non-disorder state; and (3) the detection of perturbations or abnormalities in the signal transduction pathway mediated by GALK1.

Kits for detecting expression of GALK1 in various samples, comprising at least one antibody specific to GALK1, all reagents and/or devices suitable for the detection of antibodies, the immobilization of antibodies, and the like, and instructions for using such kits in diagnosis or therapy may be produced.

A method for diagnosing a disease or disorder in a patient that is associated with alterations in GALK1 expression, may comprise: a) obtaining a biological sample from the patient; b) contacting the sample with a probe for GALK1 expression; c) comparing results from step (b) with a control; and d) determining whether step (c) indicates a likelihood of the disease or disorder. The disease may be cancer. The probe may be either DNA or protein, including an antibody.

### EXAMPLES

The following experimental section and examples are offered by way of illustration and not by way of limitation.

### I. PTEN/AKT screen

We designed a genetic screen to identify suppressors genes that when inactivated, decrease signaling through the PTEN/AKT pathway. Small interfering RNA (siRNA) libraries targetting genes from the human genome were used for these experiments. The function of individual genes was inactivated by RNAi using siRNAs designed against each gene and transfected into the human lung tumor cell line A549. The siRNA treated cells were assayed for PTEN/AKT pathway activity by monitoring changes in the amount of phosphorylated PRAS40 protein in the cytoplasm of cells (a direct AKT substrate, indicating changes in AKT activity) or the amount of phosphorylated RPS6 protein in the cytoplasm (a direct substrate of the p70S6 Kinase which is a substrate of TOR and downstream of AKT.)

Four unique individual siRNA duplexes per gene were used to knock down expression of each target. Each siRNA duplex was transfected at a final concentration of 25 nM using OligofectAmine™ lipid reagent following manufacturers' instructions (Invitrogen). A gene was scored as positive if two or more individual siRNAs reduced the amount of phosphorylated PRAS40 or RPS6 protein in A549 cells compared to negative control siRNAs. The positive result was repeated in A549 cells and a second cell line, MDA-MB-231T breast cancer cells. The reduction in phospho protein was detected and quantitated on the Cellomics® Arrayscan fluorescent microscopy platform 72 hours post transfection. The screen resulted in identification of genes that when inactivated decrease signaling through the PTEN/AKT pathway.

### II. Analysis of Table 1

The columns "GALK1 symbol", and "GALK1 name aliases " provide a symbol and the known name abbreviations for the Targets, where available, from Genbank. "GALK1 RefSeq_NA or GI_NA", "GALK1 GI_AA", "GALK1 NAME", and "GALK1 Description" provide the reference DNA sequences for the GALK1 as available from National Center for Biology Information (NCBI), GALK1 protein Genbank identifier number (GI#), GALK1 name, and GALK1 description, all available from Genbank, respectively. The length of each amino acid is in the "GALK1 Protein Length" column.

**Table 1**

| | | | | | | |
|---|---|---|---|---|---|---|
| GALK1 symbol | GALK1 name aliases | GALK1 RefSeq_NA or GI_NA | GALK1 GI_AA | GALK1 name | GALK1 description | GALK1 protein length |
| GALK1 | galactokinase 1\|GALK\|GK1\|GALK I | NM_000154 | 30582849 | galactokinase 1 | galactokinase activity; transferase activity; kinase activity; ATP binding; galactokinase activity | 392 |

### III. Kinase assay

A purified or partially purified GALK1 is diluted in a suitable reaction buffer, e.g., 50 mM Hepes, pH 7.5, containing magnesium chloride or manganese chloride (1-20 mM) and a peptide or polypeptide substrate, such as myelin basic protein or casein (1-10 µg/ml). The final concentration of the kinase is 1-20 nM. The enzyme reaction is conducted in microtiter plates to facilitate optimization of reaction conditions by increasing assay throughput. A 96-well microtiter plate is employed using a final volume 30-100 µl. The reaction is initiated by the addition of ³³P-gamma-ATP (0.5 µCi/ml) and incubated for 0.5 to 3 hours at room temperature. Negative controls are provided by the addition of EDTA, which chelates the divalent cation (Mg2⁺ or Mn²⁺) required for enzymatic activity. Following the incubation, the enzyme reaction is quenched using EDTA. Samples of the reaction are transferred to a 96-well glass fiber filter plate (MultiScreen, Millipore). The filters are subsequently washed with phosphate-buffered saline, dilute phosphoric acid (0.5%) or other suitable medium to remove excess radiolabeled ATP. Scintillation cocktail is added to the filter plate and the incorporated radioactivity is quantitated by scintillation counting (Wallac/Perkin Elmer). Activity is defmed by the amount of radioactivity detected following subtraction of the negative control reaction value (EDTA quench).

### III. High-Throughput In Vitro Fluorescence Polarization Assay

Fluorescently-labeled GALK1 peptide/substrate are added to each well of a 96-well microtiter plate, along with a test agent in a test buffer (10 mM HEPES, 10 mM NaCl, 6 mM magnesium chloride, pH 7.6). Changes in fluorescence polarization, determined by using a Fluorolite FPM-2 Fluorescence Polarization Microtiter System (Dynatech Laboratories, Inc), relative to control values indicates the test compound is a candidate modifier of GALK1 activity.

### IV. High-Throughput In Vitro Binding Assay.

³³P-labeled GALS1 peptide is added in an assay buffer (100 mM KCl, 20 mM HEPES pH 7.6, 1 mM MgCl₂, 1% glycerol, 0.5% NP-40, 50 mM betamercaptoethanol, 1 mg/ml BSA, cocktail of protease inhibitors) along with a test agent to the wells of a Neutralite-avidin coated assay plate and incubated at 25°C for 1 hour. Biotinylated substrate is then added to each well and incubated for 1 hour. Reactions are stopped by washing with PBS, and counted in a scintillation counter. Test agents that cause a difference in activity relative to control without test agent are identified as candidate PTEN/AKT modulating agents.

### V. Immunoprecipitations and Immunoblotting

For coprecipitation of transfected proteins, 3 × 10⁶ appropriate recombinant cells containing the GALK1 proteins are plated on 10-cm dishes and transfected on the following day with expression constructs. The total amount of DNA is kept constant in each transfection by adding empty vector. After 24 h, cells are collected, washed once with phosphate-buffered saline and lysed for 20 min on ice in 1 ml of lysis buffer containing 50 mM Hepes, pH 7.9, 250 mM NaCl, 20 mM - glycerophosphate, 1 mM sodium orthovanadate, 5 mM p-nitrophenyl phosphate, 2 mM dithiothreitol, protease inhibitors (complete, Roche Molecular Biochemicals), and 1% Nonidet P-40. Cellular debris is removed by centrifugation twice at 15,000 × g for 15 min. The cell lysate is incubated with 25 µl of M2 beads (Sigma) for 2 h at 4 °C with gentle rocking.

After extensive washing with lysis buffer, proteins bound to the beads are solubilized by boiling in SDS sample buffer, fractionated by SDS-polyacrylamide gel electrophoresis, transferred to polyvinylidene difluoride membrane and blotted with the indicated antibodies. The reactive bands are visualized with horseradish peroxidase coupled to the appropriate secondary antibodies and the enhanced chemiluminescence (ECL) Western blotting detection system (Amersham Pharmacia Biotech).

### VIII. Expression analysis

All cell lines used in the following experiments are NCI (National Cancer Institute) lines, and are available from ATCC (American Type Culture Collection, Manassas, VA 20110-2209). Normal and tumor tissues are obtained from Impath, UC Davis, Clontech, Stratagene, Ardais, Genome Collaborative, and Ambion.

TaqMan® analysis is used to assess expression levels of the disclosed genes in various samples.

RNA is extracted from each tissue sample using Qiagen (Valencia, CA) RNeasy kits, following manufacturer's protocols, to a final concentration of 50ng/µl. Single stranded cDNA is then synthesized by reverse transcribing the RNA samples using random hexamers and 500ng of total RNA per reaction, following protocol 4304965 of Applied Biosystems (Foster City, CA).

Primers for expression analysis using TaqMan® assay (Applied Biosystems, Foster City, CA) are prepared according to the TaqMan® protocols, and the following criteria: a) primer pairs are designed to span introns to eliminate genomic contamination, and b) each primer pair produced only one product. Expression analysis is performed using a 7900HT instrument.

TaqMan® reactions are carried out following manufacturer's protocols, in 25 µl total volume for 96-well plates and 10 µl total volume for 384-well plates, using 300nM primer and 250 nM probe, and approximately 25ng of cDNA. The standard curve for result analysis is prepared using a universal pool of human cDNA samples, which is a mixture of cDNAs from a wide variety of tissues so that the chance that a target will be present in appreciable amounts is good. The raw data are normalized using 18S rRNA (universally expressed in all tissues and cells).

For each expression analysis, tumor tissue samples are compared with matched normal tissues from the same patient. A gene is considered overexpressed in a tumor when the level of expression of the gene is 2 fold or higher in the tumor compared with its matched normal sample. In cases where normal tissue is not available, a universal pool of cDNA samples is used instead. In these cases, a gene is considered overexpressed in a tumor sample when the difference of expression levels between a tumor sample and the average of all normal samples from the same tissue type is greater than 2 times the standard deviation of all normal samples (i.e., Tumor - average(all normal samples) > 2 x STDEV(all normal samples) ). The GALK1 gene is highly expressed in colon, colon-AC, colon non-AC, head/neck, lung, lung-LCLC, ovary, pancreas, skin and stomach tumor samples relative to normal tissue samples. The GALK1 gene is underexpressed in kidney tumor samples relative to normal tissue samples.

The GALK1 gene is highly expressed in a number of cell lines including the MDA_MB_231T, breast cell line. The GALK1 gene is highly expressed in the U87_MG uterine cell line. The GALK1 gene is highly expressed in the A549 lung cell line. The GALK1 gene is highly expressed in the PC-3 prostate cell line.

A modulator identified by an assay described herein can be further validated for therapeutic effect by administration to a tumor in which the gene is overexpressed. A decrease in tumor growth confirms therapeutic utility of the modulator. Prior to treating a patient with the modulator, the likelihood that the patient will respond to treatment can be diagnosed by obtaining a tumor sample from the patient, and assaying for expression of the gene targeted by the modulator. The expression data for the gene(s) can also be used as a diagnostic marker for disease progression. The assay can be performed by expression analysis as described above, by antibody directed to the gene target, or by any other available detection method.

### VIII. Cellular assays

We performed cellular assays in mammalian cells to validate targets that were identified in a genetic screen as suppressor genes that when inactivated, decrease signaling through the AKT pathway. (see Hsieh AC et al. (2004) NAR 32(3):893-901. as a proof of principle for an siRNA screen for PTEN pathway modifiers.) The function of individual genes was inactivated by RNAi using siRNAs designed against each gene and transfected into the human tumor cell lines A549, MDA-MB231-T, and PC-3 cells. The siRNA treated cells were assayed for AKT pathway activity by monitoring changes in three relevant pathway readouts: 1) The amount of phosphorylated PRAS40 protein in the cytoplasm of cells (a direct AKT substrate, indicating changes in AKT activity); 2) the amount of phosphorylated RPS6 protein in the cytoplasm (a direct substrate of the p70S6 Kinase which is a substrate of TOR and downstream of AKT.); and 3) The amount of phosphorylation of Akt substrates as a whole by the use of an antibody which recognizes the consensus phosphorylation site in these substrates.

4 unique individual siRNA duplexes per gene were used to knock down expression of each target. Each siRNA duplex was transfected at a final concentration of 25 nM using OligofectAmine lipid reagent following manufacturer's instructions (Invitrogen). A gene was scored as positive if two or more individual siRNAs reduced the amount of phosphorylated PRAS40 or RPS6 protein or Akt substrate phosphorylation in the cell types described above compared to negative control siRNAs. The reduction in phospho protein was detected and quantified on the Cellomics Arrayscan fluorescent microscopy platform 72 hours post transfection. Positive results in these validation assays confirm that these targets, when inactivated, decrease signaling through the AKT pathway. The GALK1 siRNAs synthesized reduced the amount of Phospho-RPS6 by at least 20% in A549, PC-3 and MB231T cells. The GALK1 siRNAs synthesized reduced the amount of Phospho-Pras40 by at least 20% in PC-3 and MB231T cells. The GALK1 siRNAs synthesized reduced the amount of Phospho-Pan AKT Substrate assays by at least 20% in A549, PC-3 and MB231T cells.

In addition, these targets were validated in several cell based assays designed to look at the effect of target knockdown on phenotypic endpoints such as reduction of proliferation and induction of apoptosis.

**Apoptosis assays.** Apoptosis or programmed cell death is a suicide program is activated within the cell, leading to fragmentation of DNA, shrinkage of the cytoplasm, membrane changes and cell death. Apoptosis is mediated by proteolytic enzymes of the caspase family. Many of the altering parameters of a cell are measurable during apoptosis.

### Caspase 3 assay:

The caspase 3 assay is based on the activation of the caspase cleavage activity as part of a cascade of events that occur during programmed cell death in many apoptotic pathways. To determine if mPTENAKT pathway targets induced Caspase 3 mediated apoptosis when target activity is reduced, the cell types A549, PC-3, MDA-MB231-T and U87-MG were treated with 4 unique individual siRNA duplexes per gene to knock down expression of each target. Each siRNA duplex was transfected at a final concentration of 25 nM using OligofectAmine lipid reagent following manufacturer's instructions (Invitrogen). The detection of cleaved caspase 3, indicating an intermediate stage of apoptosis, was detected with an antibody that specifically recognizes this form of caspase 3 and quantified on the Cellomics Arrayscan fluorescent microscopy platform 72 hours post transfection. The GALK1 siRNAs synthesized increased the level of detectable cleaved caspase 3 in PC-3 cells.

### Phospho Histone H2B Assay:

The Phospho-histone H2B assay is another apoptosis assay, based on phosphorylation of histone H2B as a result of apoptosis. A fluorescent dye that is associated with phosphohistone H2B is used to measure the increase of phosphohistone H2B as a result of apoptosis. To determine if mPTENAKT pathway targets induce phosphohistone H2B mediated apoptosis when target activity is reduced, the cell types A549, PC-3, MDA-MB231-T and U87-MG were treated with 4 unique individual siRNA duplexes per gene to knock down expression of each target. Each siRNA duplex was transfected at a final concentration of 25 nM using OligofectAmine lipid reagent following manufacturer's instructions (Invitrogen). The detection of phospho histone H2B, indicating induction of apoptosis, was detected with an antibody that specifically recognizes phosphorylated histone H2B and quantified on the Cellomics Arrayscan fluorescent microscopy platform 72 hours post transfection. The GALK1 siRNAs synthesized induced the phosphorylation of histone H2B in 231T cells, and in PC-3 cells.

**Cell proliferation and cell count assays.** To determine if the PTEN pathway targets reduce cell proliferation a bromodeoxyuridine (BRDU) incorporation assay was performed. This assay identifies a cell population undergoing DNA synthesis by incorporation of BRDU into newly-synthesized DNA. Newly-synthesized DNA is then detected using an anti-BRDU antibody (Hoshino et al., 1986, Int. J. Cancer 38, 369; Campana et al., 1988, J. Immunol. Meth. 107, 79). To determine if mPTENAKT pathway targets reduce BrdU incorporation and therefore cellular proliferation when target activity is reduced, the cell types A549, PC-3, MDA-MB231-T and U87-MG were treated with 4 unique individual siRNA duplexes per gene to knock down expression of each target. Each siRNA duplex was transfected at a final concentration of 25 nM using OligofectAmine lipid reagent following manufacturer's instructions (Invitrogen). At 72 hours post-transfection, BrdU was added to the cells for 4 hours to allow incorporation. To measure whether BrdU and therefore proliferation was reduced following target inactivation, BrdU was detected with an anti-BrdU antibody and quantified on the Cellomics Arrayscan fluorescent microscopy platform. The GALK1 siRNAs synthesized decreased BrdU incorporation in 231T cells, A549 cells, U87MG cells, and PC-3 cells.

In addition, to measure if target inactivation results in reduction in cell number, the cell types A549, PC-3, MDA-MB231-T and U87-MG were treated with 4 unique individual siRNA duplexes per gene to knock down expression of each target. Each siRNA duplex was transfected at a final concentration of 25 nM using OligofectAmine lipid reagent following manufacturer's instructions (Invitrogen). At 72 hours, the Hoescht reagent was added, which incorporates into chromosomal DNA and serves to demarcate the nucleus of each individual cell. Incorporation of Hoescht was then quantified on the Cellomics Arrayscan fluorescent microscopy platform. The GALK1 siRNAs synthesized reduced the cell counts in A549 cells, MB231T cells, and PC-3 cells.

### Nucleic Acid and Polypeptide sequences

SEQ ID NO:1 
>gi|30582849|gb|AAP35651.1| galactokinase 1 [Homo
sapiens] gi|4503895|ref|NP_000145.1| galactokinase







SEQ ID NO:2 
GALK1 NucleotideTarget, Taqman, Isoform   Hsap20115930
redundant GIs: 4503894
>gi|4503894|ref|NM_000154.1| Homo sapiens galactokinase 1
(GALK1), mRNA
CCCGAGCATCCCGCGCCGACGGGGCTGTGCCGGAGCAGCTGTGCAGAGCTGCAGGCGCGC
GTCATGGCTGCTTTGAGACAGCCCCAGGTCGCGGAGCTGCTGGCCGAGGCCCGGCGAGCC
TTCCGGGAGGAGTTCGGGGCCGAGCCCGAGCTGGCCGTGTCAGCGCCGGGCCGCGTCAAC
CTCATCGGGGAACACACGGACTACAACCAGGGCCTGGTGCTGCCTATGGCTCTGGAGCTC
ATGACGGTGCTGGTGGGCAGCCCCCGCAAGGATGGGCTGGTGTCTCTCCTCACCACCTCT
GAGGGTGCCGATGAGCCCCAGCGGCTGCAGTTTCCACTGCCCACAGCCCAGCGCTCGCTG
GAGCCTGGGACTCCTCGGTGGGCCAACTATGTCAAGGGAGTGATTCAGTACTACCCAGCT
GCCCCCCTCCCTGGCTTCAGTGCAGTGGTGGTCAGCTCAGTGCCCCTGGGGGGTGGCCTG
TCCAGCTCAGCATCCTTGGAAGTGGCCACGTACACCTTCCTCCAGCAGCTCTGTCCAGAC
TCGGGCACAATAGCTGCCCGCGCCCAGGTGTGTCAGCAGGCCGAGCACAGCTTCGCAGGG
ATGCCCTGTGGCATCATGGACCAGTTCATCTCACTTATGGGACAGAAAGGCCACGCGCTG
CTCATTGACTGCAGGTCCTTGGAGACCAGCCTGGTGCCACTCTCGGACCCCAAGCTGGCC
GTGCTCATCACCAACTCTAATGTCCGCCACTCCCTGGCCTCCAGCGAGTACCCTGTGCGG
CGGCGCCAATGTGAAGAAGTGGCCCGGGCGCTGGGCAAGGAAAGCCTCCGGGAGGTACAA
CTGGAAGAGCTAGAGGCTGCCAGGGACCTGGTGAGCAAAGAGGGCTTCCGGCGGGCCCGG
CACGTGGTGGGGGAGATTCGGCGCACGGCCCAGGCAGCGGCCGCCCTGAGACGTGGCGAC
TACAGAGCCTTTGGCCGCCTCATGGTGGAGAGCCACCGCTCACTCAGAGACGACTATGAG
GTGAGCTGCCCAGAGCTGGACCAGCTGGTGGAGGCTGCGCTTGCTGTGCCTGGGGTTTAT
GGCAGCCGCATGACGGGCGGTGGCTTCGGTGGCTGCACGGTGACACTGCTGGAGGCCTCC
GCTGCTCCCCACGCCATGCGGCACATCCAGGAGCACTACGGCGGGACTGCCACCTTCTAC
 
CTCTCTCAAGCAGCCGATGGAGCCAAGGTGCTGTGCTTGTGAGGCACCCCCAGGACAGCA
CACGGTGAGGGTGCGGGGCCTGCAGGCCAGTCCCACGGCTCTGTGCCCGGTGCCATCTTC
CATATCCGGGTGCTCAATAAACTTGTGCCTCCAATGTGGTA
SEQ ID NO:3

GALK1 Isoform     Hsap20115936
redundant GIs: 34526802
>gi|34526802|dbj|AK130064.1| Homo sapiens cDNA FLJ26554 fis,
clone LNF01773, highly similar to Galact
ATCCCGCGCCGACGGGGCTGTGCCGGAGCAGCTGTGCAGAGCTGCAGGCGCGCGTCATGG
CTGCTTTGAGACAGCCCCAGGTCGCGGAGCTGCTGGCCGAGGCCCGGCGAGCCTTCCGGG
AGGAGTTCGGGGCCGAGCCCGAGCTGGCCGTGTCAGCGCCGGGCCGCGTCAACCTCATCG
GGGAACACACGGACTACAACCAGGGCCTGGTGCTGCCTATGGCTCTGGAGCTCATGACGG
TGCTGGTGGGCAGCCCCCGCAAAGAGGGCTTCCGGCGGGCCCGGCACGTGGTGGGGAGA
TTCGGCGCACGGCCCAGGCAGCGGCCGCCCTGAGACGTGGCGACTACAGAGCCTTTGGCC
GCCTCATGGTGGAGAGCCACCGCTCACTCAGAGACGACTATGAGGTGAGCTGCCCAGAGC
TGGACCAGCTGGTGGAGGCTGCGCTTGCTGTGCCTGGGGTTTATGGCAGCCGCATGACGG
GCGGTGGCTTCGGTGGCTGCACGGTGACACTGCTGGAGGCCTCCGCTGCTCCCCACGCCA
TGCGGCACATCCAGGAGCACTACGGCGGGACTGCCACCTTCTACCTCTCTCAAGCAGCCG
ATGAGCCCCAGCGGCTGCAGTTTCCACTGCCCACAGCCCAGCGCTCGCTGGAGCCTGGGA
CTCCTCGGTGGGCCAACTATGTCAAGGGAGTGATTCAGTACTACCCAGCTGCCCCCCTCC
CTGGCTTCAGTGCAGTGGTGGTCAGCTCAGTGCCCCTGGGGGGTGGCCTGTCCAGCTCAG
CATCCTTGGAAGTGGCCACGTACACCTTCCTCCAGCAGCTCTGTCCAGACTCGGGCACAA
TAGCTGCCCGCGCCCAGGTGTGTCAGCAGGCCGAGCACAGCTTCGCAGGGATGCCCTGTG
GCATCATGGACCAGTTCATCTCACTTATGGGACAGAAAGGCCACGCGCTGCTCATTGACT
GCAGGTCCTTGGAGACCAGCCTGGTGCCACTCTCGGACCCCAAGCTGGCCGTGCTCATCA
CCAACTCTAATGTCCGCCACTCCCTGGCCTCCAGCGAGTACCCTGTGCGGCGGCGCCAAT
GTGAAGAAGTGGCCCGGGCGCTGGGCAAGGAAAGCCTCCGGGAGGTACAACTGGAAGAGC
TAGAGGCTGCCAGGGACCTGGTGAGCAAAGAGGGCTTCCGGCGGGCCCGGCACGTGGTGG
TGTCTCTCCTCACCACCTCTGAGGGTGCCAAGGTGCTGTGCTTGTGAGGCACCCCCAGGA
CAGCACACGGTGAGGGTGCGGGGCCTGCAGGCCAGTCCCACGGCTCTGTGCCCGGTGCCA
TCTTCCATATCCGGGTGCTCAATAAACTTGTGCCTCC

## Claims

1. An in vitro method of identifying a candidate PTEN/AKT modulating agent, said method comprising the steps of:
(a) providing an assay system capable of detecting GALK1 expression and/or kinase activity comprising a GALK1 polypeptide or a nucleic acid encoding said polypeptide;
wherein said GALK1 polypeptide comprises:
(i) the polypeptide having the amino acid sequence shown as SEQ ID NO: 1;
(ii) a functionally active fragment of the polypeptide having the amino acid sequence shown as SEQ ID NO: 1, wherein said functionally active fragment is a kinase domain-containing fragment having galactose kinase activity and comprising at least 100 contiguous amino acids of SEQ ID NO: 1; or
(iii) a polypeptide which has at least 80% sequence identity with SEQ ID NO: 1 and has galactose kinase activity;
(b) contacting the assay system with a test agent; and
(c) determining the expression or kinase activity of GALK1 in the assay system, wherein a change in GALK1 expression or kinase activity between the presence and absence of said test agent identifies the test agent as a candidate PTEN/AKT modulating agent.

2. The method of Claim 1 wherein the assay system comprises cultured cells that express the GALK1 polypeptide.

3. The method of Claim 2 wherein the cultured cells additionally have defective PTEN/AKT function.

4. The method of Claim 1 wherein the assay system includes a screening assay comprising the GALK1 polypeptide, and the candidate test agent is a small molecule modulator.

5. The method of Claim 4 wherein the assay is a binding assay.

6. The method of Claim 1 wherein the assay system is selected from the group consisting of an apoptosis assay system, a cell proliferation assay system, an angiogenesis assay system, and a hypoxic induction assay system.

7. The method of Claim 1 wherein the assay system includes a binding assay comprising the GALK1 polypeptide and the candidate test agent is an antibody.

8. The method of Claim 1 wherein the assay system includes an expression assay comprising the GALK1 nucleic acid and the candidate test agent is a nucleic acid modulator.

9. The method of claim 8 wherein the nucleic acid modulator is an antisense oligomer.

10. The method of Claim 8 wherein the nucleic acid modulator is a phosphothioate morpholino oligomer (PMO).

11. The method of Claim 1 additionally comprising:
(d) administering the test agent identified in (c) to an in vitro model system comprising cells defective in PTEN/AKT function and, detecting a phenotypic change in the model system that indicates that the PTEN/AKT function is restored.

12. The method of Claim 1, comprising the additional steps of:
(d) providing an in vitro secondary assay system comprising cultured cells expressing GALK1,
(e) contacting the secondary assay system with the test agent of (b) or an agent derived therefrom under conditions whereby, but for the presence of the test agent or agent derived therefrom, the system provides a reference activity; and
(f) detecting an agent-biased activity of the secondary assay system, wherein a difference between the agent-biased activity and the reference activity of the secondary assay system confirms the test agent or agent derived therefrom as a candidate PTEN/AKT pathway modulating agent,
and wherein the secondary assay detects an agent-biased change in the PTEN/AKT pathway.

## Patentansprüche

1. Ein In-vitro-Verfahren zum Identifizieren eines PTEN/AKT modulierenden Wirkstoffkandidaten, wobei das Verfahren die folgenden Schritte beinhaltet:
(a) Bereitstellen eines Assaysystems, das in der Lage ist, GALK1-Expression und/oder -Kinaseaktivität zu erkennen, und das ein GALK1-Polypepid oder eine Nukleinsäure, die das Polypeptid codiert, beinhaltet;
wobei das GALK1-Polypeptid Folgendes beinhaltet:
(i) das Polypeptid mit der Aminosäuresequenz, die als SEQ ID NO:1 gezeigt ist;
(ii) ein funktionell aktives Fragment des Polypeptids mit der Aminosäuresequenz, die als SEQ ID NO:1 gezeigt ist, wobei das funktionell aktive Fragment ein eine Kinasedomäne enthaltendes Fragment ist, das eine Galaktosekinaseaktivität aufweist und mindestens 100 zusammenhängende Aminosäuren von SEQ ID NO:1 beinhaltet; oder
(iii) ein Polypeptid, das eine Sequenzidentität mit SEQ ID NO:1 von mindestens 80 % aufweist und Galaktosekinaseaktivität aufweist;
(b) In-Kontakt-Bringen des Assaysystems mit einem Testwirkstoff und
(c) Bestimmen der Expression oder Kinaseaktivität von GALK1 in dem Assaysystem, wobei eine Änderung der GALK1-Expression oder -Kinaseaktivität zwischen dem Vorhandensein und der Abwesenheit des Testwirkstoffs den Testwirkstoff als einen PTEN/AKT modulierenden Wirkstoffkandidaten identifiziert.

2. Verfahren gemäß Anspruch 1, wobei das Assaysystem kultivierte Zellen beinhaltet, die das GALK1-Polypeptid exprimieren.

3. Verfahren gemäß Anspruch 2, wobei die kultivierten Zellen zusätzlich eine fehlerhafte PTEN/AKT-Funktion aufweisen.

4. Verfahren gemäß Anspruch 1, wobei das Assaysystem einen Screening-Assay umfasst, der das GALK1-Polypeptid beinhaltet, und der Testwirkstoffkandidat ein Modulator aus einem kleinen Molekül ist.

5. Verfahren gemäß Anspruch 4, wobei der Assay ein Bindungsassay ist.

6. Verfahren gemäß Anspruch 1, wobei das Assaysystem aus der Gruppe, bestehend aus einem Apoptose-Assaysystem, einem Zellproliferations-Assaysystem, einem Angiogenese-Assaysystem und einem Hypoxieinduktions-Assaysystem, ausgewählt ist.

7. Verfahren gemäß Anspruch 1, wobei das Assaysystem einen Bindungsassay umfasst, der das GALK1-Polypeptid beinhaltet, und der Testwirkstoffkandidat ein Antikörper ist.

8. Verfahren gemäß Anspruch 1, wobei das Assaysystem einen Expressionsassay umfasst, der die GALK1-Nukleinsäure beinhaltet, und der Testwirkstoffkandidat ein Modulator aus Nukleinsäure ist.

9. Verfahren gemäß Anspruch 8, wobei der Modulator aus Nukleinsäure ein Antisense-Oligomer ist.

10. Verfahren gemäß Anspruch 8, wobei der Modulator aus Nukleinsäure ein Phosphothioatmorpholino-Oligomer (PMO) ist.

11. Verfahren gemäß Anspruch 1, das zusätzlich Folgendes beinhaltet:
(d) Verabreichen des in (c) identifizierten Testwirkstoffs an ein In-vitro-Modellsystem, das hinsichtlich der PTEN/AKT-Funktion fehlerhafte Zellen beinhaltet, und Erkennen einer phänotypischen Änderung in dem Modellsystem, die anzeigt, dass die PTEN/AKT-Funktion wiederhergestellt ist.

12. Verfahren gemäß Anspruch 1, das die folgenden zusätzlichen Schritte beinhaltet:
(d) Bereitstellen eines sekundären In-vitro-Assaysystems, das die kultivierten Zellen, welche GALK1 exprimieren, beinhaltet,
(e) In-Kontakt-Bringen des sekundären Assaysystems mit dem Testwirkstoff aus (b) oder einem davon abgeleiteten Wirkstoff unter Bedingungen, wobei bei fehlendem Vorhandensein des Testwirkstoffs oder des davon abgeleiteten Wirkstoffs das System eine Referenzaktivität bereitstellt; und
(f) Erkennen einer durch den Wirkstoff verzerrten Aktivität des sekundären Assaysystems,
wobei ein Unterschied zwischen der durch den Wirkstoff verzerrten Aktivität und der Referenzaktivität des sekundären Assaysystems bestätigt, dass der Testwirkstoff oder der davon abgeleitete Wirkstoff ein den PTEN/AKT-Weg modulierender Wirkstoffkandidat ist,
und wobei der sekundäre Assay eine durch den Wirkstoff verzerrte Änderung des PTEN/AKT-Wegs erkennt.

## Revendications

1. Une méthode in vitro pour identifier un agent modulant PTEN/AKT candidat, ladite méthode comprenant les étapes consistant à :
(a) apporter un système d'épreuve capable de détecter l'expression de GALK1 et / ou son activité kinase comprenant un polypeptide GALK1 ou un acide nucléique codant pour ledit polypeptide ;
où ledit polypeptide GALK1 comprend :
(i) le polypeptide ayant la séquence d'acides aminés montrée en tant que SEQ ID NO : 1 ;
(ii) un fragment fonctionnellement actif du polypeptide ayant la séquence d'acides aminés montrée en tant que SEQ ID NO : 1, où ledit fragment fonctionnellement actif est un fragment contenant le domaine kinase présentant une activité galactokinase et comprenant au moins 100 acides aminés contigus de SEQ ID NO : 1 ; ou
(iii) un polypeptide qui présente une identité de séquence d'au moins 80 % avec SEQ ID NO : 1 et a une activité galactokinase ;
(b) mettre en contact le système d'épreuve avec un agent de test ; et
(c) déterminer l'expression ou l'activité kinase de GALK1 dans le système d'épreuve, où un changement dans l'expression de GALK1 ou dans son activité kinase entre la présence et l'absence dudit agent de test identifie l'agent de test comme étant un agent modulant PTEN/AKT candidat.

2. La méthode de la revendication 1 où le système d'épreuve comprend des cellules cultivées qui expriment le polypeptide GALK1.

3. La méthode de la revendication 2 où les cellules cultivées ont de plus une fonction PTEN/AKT défectueuse.

4. La méthode de la revendication 1 où le système d'épreuve comporte une épreuve de criblage comprenant le polypeptide GALK1, et l'agent de test candidat est un modulateur à petites molécules.

5. La méthode de la revendication 4 où l'épreuve est une épreuve de liaison.

6. La méthode de la revendication 1 où le système d'épreuve est sélectionné dans le groupe consistant en un système d'épreuve d'apoptose, un système d'épreuve de prolifération cellulaire, un système d'épreuve d'angiogenèse, et un système d'épreuve d'induction hypoxique.

7. La méthode de la revendication 1 où le système d'épreuve comporte une épreuve de liaison comprenant le polypeptide GALK1 et l'agent de test candidat est un anticorps.

8. La méthode de la revendication 1 où le système d'épreuve comporte une épreuve d'expression comprenant l'acide nucléique de GALK1 et l'agent de test candidat est un acide nucléique modulateur.

9. La méthode de la revendication 8 où l'acide nucléique modulateur est un oligomère antisens.

10. La méthode de la revendication 8 où l'acide nucléique modulateur est un oligomère morpholino phosphothioate (PMO).

11. La méthode de la revendication 1 comprenant de plus :
(d) administrer l'agent de test identifié à (c) à un système modèle in vitro comprenant des cellules défectueuses en terme de fonction PTEN/AKT et, détecter un changement phénotypique dans le système modèle qui indique que la fonction PTEN/AKT est restaurée.

12. La méthode de la revendication 1, comprenant les étapes supplémentaires consistant à :
(d) apporter un système d'épreuve secondaire in vitro comprenant des cellules en culture exprimant GALK1 ;
(e) mettre en contact le système d'épreuve secondaire avec l'agent de test de (b) ou un agent dérivé de celui-ci dans des conditions où, en l'absence de l'agent de test ou de l'agent dérivé de celui-ci, le système apporte une activité de référence ; et
(f) détecter une activité influencée par l'agent du système d'épreuve secondaire, où une différence entre l'activité influencée par l'agent et l'activité de référence du système d'épreuve secondaire confirme que l'agent de test ou l'agent dérivé de celui-ci est un agent modulant la voie PTEN/AKT candidat,
et où l'épreuve secondaire détecte un changement influencé par l'agent dans la voie PTEN/AKT.
